# EUROPEAN PATENT APPLICATION

(11) **EP 3 831 850 A1**
(43) Date of publication of application: **09.06.2021**
(21) Application number: 19814717.5
(22) Date of filing: 06.06.2019
(51) Int. Cl.: C07K 16/28, A61K 39/00, C12N 5/10, C12N 15/06, A61P 35/00

(54) **ANTIBODIES TO PROGRAMMED DEATH LIGAND (PD-L1) AND APPLICATION THEREOF**

(30) Priority: 06.06.2018 CN 201810575543
(71) Applicant: BioRay Pharmaceutical Co., Ltd., Jiaojiang District, Taizhou City, Zhejiang (CN)
(72) Inventor: WANG, Haibin, Taizhou, Zhejiang 318000 (CN); HU, Feng, Taizhou, Zhejiang 318000 (CN); ZHANG, Xuan, Taizhou, Zhejiang 318000 (CN); JIAO, Jingyu, Taizhou, Zhejiang 318000 (CN); WU, Zhenhua, Taizhou, Zhejiang 318000 (CN); GAO, Dong, Taizhou, Zhejiang 318000 (CN)
(74) Representative: Schnappauf, Georg
(86) International application number: PCT/CN2019/090228
(87) International publication number: WO 2019/233462

(57) **Abstract**

Disclosed in the present invention are antibodies to programmed death ligand (PD-L1) and an application thereof, and in particular, an application thereof in treating and/or preventing PD-L1 related conditions.

## Description

The present application claims priority of Chinese patent application No. 201810575543.4, filed on June 6, 2018, with the title of ANTIBODIES TO PROGRAMMED DEATH LIGAND (PD-L1) AND APPLICATION THEREOF, the contents of which are incorporated herein by reference in entirety.

### Technical Field

The present application relates to the bio-medical field and specifically discloses antibodies against programmed death ligand (PD-L1) and applications thereof, particularly in the treatment and/or prevention of PD-L1 related diseases.

### Background

Programmed death-1 (PD-1) is a member of the CD28 receptor family, which includes CD28, CTLA-4, ICOS, PD-1 and BTLA. The first members of this family, CD28 and ICOS, were discovered by the function of enhancing T cell proliferation after addition of monoclonal antibodies. Two cell surface glycoprotein ligands for PD-1, PD-L1 and PD-L2, have been identified. It has been shown that after binding to PD-1, they down-regulate T cell activation and cytokine secretion. PD-L1 (B7-H1) and PD-L2 (B7-DC) are B7 homologues which can bind to PD-1 but do not bind to other CD28 family members.

PD-L1 expression has been found in several mouse and human cancers, including human lung cancer, ovarian cancer, colon cancer, melanoma and various myeloma. Results have shown that PD-L1, which is highly expressed by tumor cells, can increase T cells apoptosis whereby playing an important role in the immune escape of tumors. The researchers found that the P815 tumor cell line transfected with PD-L1 gene can resist the lysis of specific CTL *in vitro,* and after inoculation in mice, it has stronger tumorigenicity and invasiveness. These biological characteristics can be reversed by blocking PD-L1. In PD-1 gene knockout mice, the PD-L1/PD-1 pathway is blocked, and inoculated tumor cells cannot form tumors. It has also been suggested that PD-L1 may be related to intestinal mucosal inflammation, and PD-L1 inhibition prevents atrophic disease related with colitis.

Some antibodies against PD-L1 have been developed in the prior art, such as MDX-1105 (WO2007/005874), Tecentriq (Atezolizumab), etc. However, there is still a need in the art for anti-PD-Ll antibodies that can bind to PD-L1 with high affinity and can block the binding of PD-1 with PD-L1.

### Summary

In an aspect, provided is an antibody against PD-L1 (PD-L1 antibody) or an antigen-binding fragment thereof, wherein the antibody or the antigen-binding fragment thereof specifically recognizes and binds to PD-L1.

In an embodiment, the PD-L1 antibody comprises Anti-PD-Ll Antibody B10, Anti-PD-Ll Antibody B11, Anti-PD-Ll Antibody H12 or a combination thereof, particularly PD-L1 Antibody B11.

In an embodiment, the antibody or the antigen-binding fragment thereof can specifically recognizes and binds to PD-L1, wherein the affinity is 1×10⁻⁹ M or less, preferably 5×10⁻¹⁰ M or less, more preferably 1×10⁻¹⁰ M or less.

In an aspect, provided is an Antibody B10 against PD-L1 (also known as PD-L1 Antibody B10 or Antibody B10 herein) or an antigen-binding fragment thereof,
wherein the antibody comprises a light chain variable region and a heavy chain variable
region, wherein
the heavy chain variable region comprises:
H CDR1, comprising an amino acid sequence as shown in SEQ ID NO:1,
H CDR2, comprising an amino acid sequence as shown in SEQ ID NO:2, and
H CDR3, comprising an amino acid sequence as shown in SEQ ID NO:3;
the light chain variable region comprises:
L CDR1, comprising an amino acid sequence as shown in SEQ ID NO:5,
L CDR2, comprising an amino acid sequence as shown in SEQ ID NO:6, and
L CDR3, comprising an amino acid sequence as shown in SEQ ID NO:7.

In an embodiment, the PD-L1 Antibody B10 comprises a heavy chain variable region, wherein the heavy chain variable region comprises an amino acid sequence as shown in SEQ ID NO:4 or an amino acid sequence having at least 85%, at least 90%, at least 95% or more sequence identity to SEQ ID NO:4.

In another embodiment, the PD-L1 Antibody B10 comprises a light chain variable region, wherein the light chain variable region comprises an amino acid sequence as shown in SEQ ID NO:8 or an amino acid sequence having at least 85%, at least 90%, at least 95% or more sequence identity to SEQ ID NO:8.

In another aspect, provided is an Antibody B11 against PD-L1 (also known as PD-L1 Antibody B11 or Antibody B11 herein) or an antigen-binding fragment thereof,
wherein the antibody comprises a light chain variable region and a heavy chain variable region, wherein
the heavy chain variable region comprises:
H CDR1, comprising an amino acid sequence as shown in SEQ ID NO:9,
H CDR2, comprising an amino acid sequence as shown in SEQ ID NO:10, and
H CDR3, comprising an amino acid sequence as shown in SEQ ID NO:11;
the light chain variable region comprises:
L CDR1, comprising an amino acid sequence as shown in SEQ ID NO:13,
L CDR2, comprising an amino acid sequence as shown in SEQ ID NO:14, and
L CDR3, comprising an amino acid sequence as shown in SEQ ID NO:15.

In an embodiment, the PD-L1 Antibody B11 comprises a heavy chain variable region, wherein the heavy chain variable region comprises an amino acid sequence as shown in SEQ ID NO:12 or an amino acid sequence having at least 85%, at least 90%, at least 95% or more sequence identity to SEQ ID NO:12.

In another embodiment, the PD-L1 Antibody B11 comprises a light chain variable region, wherein the light chain variable region comprises an amino acid sequence as shown in SEQ ID NO:16 or an amino acid sequence having at least 85%, at least 90%, at least 95% or more sequence identity to SEQ ID NO:16.

In another embodiment, the PD-L1 Antibody B11 comprises a heavy chain, wherein the heavy chain comprises an amino acid sequence as shown in SEQ ID NO:28 or an amino acid sequence having at least 85%, at least 90%, at least 95% or more sequence identity to SEQ ID NO:28.

In another embodiment, the PD-L1 Antibody B11 comprises a light chain, wherein the light chain comprises an amino acid sequence as shown in SEQ ID NO:27 or an amino acid sequence having at least 85%, at least 90%, at least 95% or more sequence identity to SEQ ID NO:27.

In another aspect, provided is an Antibody H12 against PD-L1 (also known as PD-L1 Antibody H12 or Antibody H12 herein) or an antigen-binding fragment thereof,
wherein the antibody comprises a light chain variable region and a heavy chain variable
region, wherein
the heavy chain variable region comprises:
H CDR1, comprising an amino acid sequence as shown in SEQ ID NO:17,
H CDR2, comprising an amino acid sequence as shown in SEQ ID NO:18, and
H CDR3, comprising an amino acid sequence as shown in SEQ ID NO:19;
the light chain variable region comprises:
L CDR1, comprising an amino acid sequence as shown in SEQ ID NO:21,
L CDR2, comprising an amino acid sequence as shown in SEQ ID NO:22, and
L CDR3, comprising an amino acid sequence as shown in SEQ ID NO:23.

In another embodiment, the PD-L1 Antibody H12 comprises a heavy chain variable region, wherein the heavy chain variable region comprises an amino acid sequence as shown in SEQ ID NO:20 or an amino acid sequence having at least 85%, at least 90%, at least 95% or more sequence identity to SEQ ID NO:20.

In another embodiment, the PD-L1 Antibody H12 comprises a light chain variable region, wherein the light chain variable region comprises an amino acid sequence as shown in SEQ ID NO:24 or an amino acid sequence having at least 85%, at least 90%, at least 95% or more sequence identity to SEQ ID NO:24.

In another aspect, provided is a pharmaceutical composition, comprising the antibody according to the present invention or the antigen-binding fragment thereof and a pharmaceutically acceptable carrier. In an embodiment, the antibody comprises PD-L1 Antibody B10, PD-L1 Antibody B11, PD-L1 Antibody H12 or a combination thereof, particularly PD-L1 Antibody B11.

In an aspect, provided is use of the antibody or the antigen-binding fragment thereof according to the invention or the pharmaceutical composition according to the invention for the manufacture of a medicament for treating a neoplastic disease.

In another aspect, provided is a method for treating a neoplastic disease, comprising administering a subject in need thereof the antibody or the antigen-binding fragment thereof according to the invention or the pharmaceutical composition according to the invention.

In another aspect, provided is the antibody or the antigen-binding fragment thereof according to the invention or the pharmaceutical composition according to the invention, for use in treating a neoplastic disease.

In an embodiment, the above-mentioned antibody comprises PD-L1 Antibody B10, PD-L1 Antibody B11, PD-L1 Antibody H12 or a combination thereof, particularly PD-L1 Antibody B11.

In yet another embodiment, the neoplastic disease is a PD-L1-positive tumor. In another embodiment, the PD-L1-positive tumor is lung cancer, ovarian cancer, colorectal cancer, melanoma or myeloma, particularly colorectal cancer or non-small cell lung cancer.

In some embodiments, the above method or use further comprises administration of other anti-tumor therapy, for example, administration of a chemotherapeutic agent, an antibody targeting other tumor-specific antigen or radiotherapy.

In another aspect, provided is an isolated nucleic acid molecule, encoding the antibody or the antigen-binding fragment thereof according to the invention. In another aspect, provided is an expression vector, comprising the nucleic acid molecule according to the invention. In another aspect, provided is a host cell, which is transformed with the nucleic acid molecule according to the invention or the expression vector according to the invention.

### Brief Description of the Drawings

Fig. 1: Binding curve of candidate antibodies.
Fig. 2: ELISA results of Antibody B10, B11 and H12.
Fig. 3: Results of activation of T cells, increase of IPNγ and IL-2 expression levels by Antibody B10, B11, H12.
Fig. 4: Results of blocking of the binding between hPD-L1 and hPD-1 by Antibody B11.
Fig. 5: Results of blocking of the interaction between CD80 and PD-L1 by Antibody B11.
Fig. 6: Results of cell biological activity of Antibody B11.
Fig. 7: Results of inhibition of tumor growth by Antibody B11.
Fig. 8: Results of inhibition of tumor activity by Antibody B11.

### Detailed Description

### Definition

In the present context, unless otherwise specified, the scientific and technical terms used herein have meanings commonly understood by a person skilled in the art. Moreover, the protein and nucleic acid chemistry, molecular biology, cell and tissue culture, microbiology, immunology-related terms and laboratory procedures used herein are terms and routine procedures widely used in the field. At the same time, in order to better understand the invention, the following definitions and explanations are provided.

As used herein, "at least one" or "one or more" can mean 1, 2, 3, 4, 5, 6, 7, 8 or more.

As used herein, the term "antibody" refers to immunoglobulin and immunoglobulin fragment, whether natural or partially or wholly synthetically (e.g., recombinant) produced, comprising any fragment comprising at least part of the variable region of an immunoglobulin molecule and retaining the binding specificity of the full-length immunoglobulin. Therefore, an antibody includes any protein having a binding domain which is homologous or substantially homologous to an immunoglobulin antigen-binding domain (antibody binding site). An antibody encompasses an antibody fragment. As used herein, the term antibody comprises synthetic antibody, recombinant antibody, multispecific antibody (e.g. bispecific antibody), human antibody, non-human antibody, humanized antibody, chimeric antibody, intracellular antibody and antibody fragment, such as but not limited to Fab fragment, Fab' fragment, F(ab')₂ fragment, Fv fragment, disulfide-linked Fv (dsFv), Fd fragment, Fd' fragment, single-chain Fv (scFv), single-chain Fab (scFab), diabody, anti-idiotype (anti-Id) antibody, or antigen-binding fragment of any of the above antibodies. The antibodies provided herein include any immunoglobulin type (e.g., IgG, IgM, IgD, IgE, IgA and IgY), any class (e.g. IgG1, IgG2, IgG3, IgG4, IgA1 and IgA2) or subclass (e.g., IgG2a And IgG2b) members. In a preferable embodiment, the antibody according to the invention is human antibody.

As used herein, an "antibody fragment" or "antigen-binding fragment" of an antibody refers to any part of a full-length antibody, which is shorter than the full length, but at least comprises part of the variable region of the antibody binding to the antigen (such as one or more CDR and/or one or more antibody binding sites), and therefore retains the binding specificity and at least part of the specific binding ability of the full-length antibody. Therefore, an antigen-binding fragment refers to an antibody fragment which comprises the antigen-binding part binding to the same antigen as the antibody from which the antibody fragment is derived. An antibody fragment includes an antibody derivative produced by enzymatic treatment of a full-length antibody, and synthetically produced derivative, such as recombinantly produced derivative. An antibody includes antibody fragment. Examples of antibody fragments include but are not limited to Fab, Fab', F(ab')₂, single-chain Fv (scFv), Fv, dsFv, diabody, Fd and Fd' fragments and other fragments, including modified fragments (see, e.g. Methods in Molecular Biology, Vol 207: Recombinant Antibodies for Cancer Therapy Methods and Protocols (2003); Chapter 1; p 3-25, Kipriyanov). The fragment may include multiple chains linked together, for example, by disulfide bond and/or by peptide linker. An antibody fragment generally contains at least or about 50 amino acids, and typically at least or about 200 amino acids. An antigen-binding fragment includes any antibody fragment which when inserted into the antibody framework (for example, by replacing a corresponding region), obtains an antibody that immuno-specifically binds to the antigen.

As used herein, a "conventional antibody" refers to an antibody which includes two heavy chains (which can be designated as H and H') and two light chains (which can be designated as L and L') and two antigen-binding sites, where each heavy chain may be a full-length immunoglobulin heavy chain or any functional region thereof that retains antigen-binding ability (for example, the heavy chain includes but is not limited to V_{H} chain, V_{H}-C_{H}1 chain and V_{H}-C_{H}1-C_{H}2-C_{H}3 chain), and each light chain may be a full-length light chain or any functional region (e.g., a light chain includes but is not limited to V_{L} chain and V_{L}-C_{L} chain). Each heavy chain (H and H') is paired with a light chain (L and L', respectively).

As used herein, a full-length antibody refers to an antibody which has two full-length heavy chains (e.g. V_{H}-C_{H}1-C_{H}2-C_{H}3 or V_{H}-C_{H}1-C_{H}2-C_{H}3-C_{H}4) and two full-length light chains (V_{L}-C_{L}) and a hinge region. For example, an antibody produced naturally by antibody-secreting B cells and a synthetically produced antibody with the same domains.

As used herein, a dsFv refers to an Fv with engineered intramolecular disulfide bonds that stabilize the V_{H}-V_{L} pair.

As used herein, a Fab fragment is an antibody fragment obtained by digesting a full-length immunoglobulin with papain, or, for example a fragment with the same structure synthesized by a recombinant method. A Fab fragment contains a light chain (comprising V_{L} and C_{L}) and another chain containing a variable domain of the heavy chain (V_{H}) and a constant region domain of the heavy chain (C_{H}1).

As used herein, a F(ab')₂ fragment is an antibody fragment obtained from pepsin digestion of immunoglobulins at pH 4.0-4.5, or, for example a fragment with the same structure synthesized by a recombinant method. A F(ab')₂ fragment basically contains two Fab fragments, where each heavy chain part contains several extra amino acids, including cysteines that form disulfide bond connecting the two fragments.

As used herein, a Fab' fragment is a fragment containing half of the F(ab')₂ fragment (a heavy chain and a light chain).

As used herein, an scFv fragment refers to an antibody fragment comprising a variable light chain (V_{L}) and a variable heavy chain (V_{H}) covalently connected in any order via a polypeptide linker. The length of the linker allows substantially non-interfering bridging of the two variable domains. Exemplary linker is (Gly-Ser)ₙ residues dispersed with some Glu or Lys residues to increase solubility.

As used herein, a variable domain or variable region is a specific Ig domain of an antibody heavy chain or light chain, which comprises amino acid sequences that vary between different antibodies. Each light chain and each heavy chain have a variable region domain V_{L} and V_{H}, respectively. Variable domains provide antigen specificity and therefore are responsible for antigen recognition. Each variable region comprises CDR and framework region (FR), and CDR is part of the antigen-binding site domain.

As used herein, "antigen-binding domain" and "antigen-binding site" are used synonymously to refer to the domain within the antibody that recognizes and physically interacts with the cognate antigen. A natural conventional full-length antibody molecule has two conventional antigen-binding sites, each containing a heavy chain variable region part and a light chain variable region part. The conventional antigen-binding site contains a loop that connects the antiparallel β chains in the variable region domain. The antigen-binding site can contain other parts of the variable region domain. Each conventional antigen-binding site contains 3 hypervariable regions from the heavy chain and 3 hypervariable regions from the light chain. The hypervariable region is also called complementarity determining region (CDR).

As used herein, "hypervariable region", "HV", "complementarity determining region" and "CDR" and "antibody CDR" can be used interchangeably and refer to each of multiple parts in the variable regions that together form the antigen-binding site of the antibody. Each variable region domain contains 3 CDRs, designated as CDR1, CDR2 and CDR3. For example, a light chain variable region domain contains 3 CDRs, designated as L CDR1 (or VL CDR1), L CDR2 (or VL CDR2) and L CDR3 (or VL CDR3); a heavy chain variable region domain contains 3 CDRs, designated as H CDR1 (or VH CDR1), H CDR2 (or VH CDR2) and H CDR3 (or VH CDR3). The 3 CDRs in the variable region are discontinuous along the linear amino acid sequence but are close in the folded polypeptide. The CDRs are located in the loop connecting the parallel chains of β sheets of the variable domains. As described herein, a person skilled in the art knows and can identify CDRs based on Kabat or Chothia numbering (see, for example, Kabat, E.A. et al. (1991) Sequences of Proteins of Immunological Interest, Fifth Edition, U.S. Department of Health and Human Services, NIH Publication No. 91-3242, and Chothia, C. et al. (1987) J. Mol. Biol. 196:901-917).

As used herein, a framework region (FR) is a structural domain within the antibody variable region domain located in the β sheet. In terms of amino acid sequence, the FR region is relatively more conservative than the hypervariable region.

As used herein, a "constant region" domain is a domain in the antibody heavy chain or light chain, which contains an amino acid sequence that is relatively more conserved than the amino acid sequence of the variable region domain. In a conventional full-length antibody molecule, each light chain has a single light chain constant region (C_{L}) domain, and each heavy chain contains one or more heavy chain constant region (C_{H}) domains, including C_{H}1, C_{H}2, C_{H}3 and C_{H}4. The full-length IgA, IgD and IgG isotypes contain C_{H}1, C_{H}2, C_{H}3 and the hinge region, while IgE and IgM contain C_{H}1, C_{H}2, C_{H}3 and C_{H}4. The C_{H}1 and C_{L} domains extend the Fab arm of the antibody molecule, and therefore are helpful for interacting with antigens and turning the antibody arm. The antibody constant region can serve for effector functions, such as but not limited to removing antigens, pathogens and toxins specifically bound by the antibody, for example by interacting with various cells, biological molecules and tissues.

As used herein, a functional region of the V_{H} domain is at least part of the complete V_{H} domain that retains at least part of the binding specificity of the complete V_{H} domain (e.g., by retaining one or more CDRs of the complete V_{H} domain), such that the functional region of the V_{H} domain alone or in combination with another antibody domain (such as a V_{L} domain) or a region thereof binds to an antigen. Exemplary functional region of the V_{H} domain is a region comprising CDR1, CDR2 and/or CDR3 of the V_{H} domain.

As used herein, a functional region of the V_{L} domain is at least part of the complete V_{L} domain that retains at least part of the binding specificity of the complete V_{L} domain (e.g., by retaining one or more CDRs of the complete V_{L} domain), such that the functional region of the V_{L} domain alone or in combination with another antibody domain (such as a V_{H} domain) or a region thereof binds to an antigen. Exemplary functional region of the V_{L} domain is a region comprising CDR1, CDR2 and/or CDR3 of the V_{L} domain.

"Affinity" or "binding affinity" KD is often determined by measuring the equilibrium association constant (ka) and equilibrium dissociation constant (kd) and calculating the quotient of kd divided by ka (KD = kd/ka). As used herein, "specific binding" or "immune-specific binding" with reference to an antibody or an antigen-binding fragment thereof is used interchangeably herein and refers to the ability of the antibody or the antigen-binding fragment to form one or more non-covalent bonds with the cognate antigen through non-covalent interaction between the antibody and the antibody-binding site of the antigen. The antigen can be an isolated antigen or present in, for example, tumor cells.

In an embodiment according to the invention, the antibody or the antigen-binding fragment thereof according to the invention can bind to the target antigen with an affinity of 2×10⁻⁹M or lower, 1×10⁻⁹M or lower, 9×10⁻¹⁰M or lower, 8×10⁻¹⁰M or lower, 7×10⁻¹⁰M or lower, 6×10⁻¹⁰M or lower, 5×10⁻¹⁰M or lower, 4×10⁻¹⁰M or lower, 3×10⁻¹⁰M or lower, 2×10⁻¹⁰M or lower, 1×10⁻¹⁰M or lower (e.g. PD-L1, such as human PD-L1).

Affinity can be easily determined using conventional techniques, such as by equilibrium dialysis; by using the BIAcore 2000 instrument, using the general methods provided by the manufacturer; by using radiolabeled target antigen for radioimmunoassay; or by other methods known to a person skilled in the art.

An "isolated protein", "isolated polypeptide" or "isolated antibody" means that the protein, polypeptide or antibody, (1) is not associated with its naturally related components in its natural state, (2) does not contain other protein from the same species, (3) is expressed by cells from different species, or (4) does not exist in nature. Therefore, a chemically synthesized polypeptide or a polypeptide synthesized in a cell system different from the natural source of the polypeptide will be "separated" from the naturally related components. It can also be isolated such that the protein is substantially free of the natural related components, that is, using protein purification techniques well known in the art.

In a peptide or protein, suitable conservative amino acid substitutions are known to a person skilled in the art and can generally be performed without changing the biological activity of the resulting molecule. Generally, a person skilled in the art will recognize a single amino acid substitution in a non-essential region of a polypeptide will not substantially change the biological activity (see, e.g. Watson et al., Molecular Biology of the Gene, 4th Edition, 1987, The Benjamin/Cummings Pub. co., p.224).

As used herein, the terms "polynucleotide" and "nucleic acid molecule" refer to an oligomer or polymer comprising at least two linked nucleotides or nucleotide derivatives, including deoxyribonucleic acid (DNA) and ribonucleic acid (RNA) which are generally linked by phosphodiester bonds.

As used herein, an isolated nucleic acid molecule is a nucleic acid molecule which is separated from other nucleic acid molecules present in the natural source of the nucleic acid molecule. An "isolated" nucleic acid molecule such as a cDNA molecule can be prepared by recombinant technology and is substantially free of other cellular materials or culture media, or is substantially free of chemical precursors or other chemical components during chemical synthesis. The exemplary isolated nucleic acid molecule provided herein includes the isolated nucleic acid molecule encoding the provided antibody or antigen-binding fragment.

Sequence "identity" has a recognized meaning in the art, and the percentage of sequence identity between two nucleic acid or polypeptide molecules or regions can be calculated using published techniques. The sequence identity can be determined along the full length of the polynucleotide or polypeptide or along the region of the molecule (see, e.g.: Computational Molecular Biology, Lesk, A.M., ed., Oxford University Press, New York, 1988; Biocomputing: Informatics and Genome Projects, Smith, D.W., ed., Academic Press, New York, 1993; Computer Analysis of Sequence Data, Part I, Griffin, A.M., and Griffin, H.G., eds., Humana Press, New Jersey, 1994; Sequence Analysis in Molecular Biology, von Heinje, G., Academic Press, 1987; and Sequence Analysis Primer, Gribskov, M. and Devereux, J., eds., M Stockton Press, New York, 1991). Although there are many methods for determing the identity between two polynucleotides or polypeptides, the term "identity" is well known to a person skilled in the art (Carrillo, H. & Lipman, D., SIAM J Applied Math 48:1073 (1988)).

As used herein, the term "expression" refers to the process of producing polypeptides through the transcription and translation of polynucleotides. The expression level of polypeptides can be evaluated by any method known in the art, including, for example, methods of measuring the amount of polypeptide produced from a host cell. Such methods can include but are not limited to quantification of polypeptides in cell lysates by ELISA, Coomassie blue staining after gel electrophoresis, Lowry protein assay and Bradford protein assay.

As used herein, a "host cell" is a cell used to receive, maintain, replicate and amplify a vector. A host cell can also be used to express the polypeptide encoded by the vector. When the host cell divides, the nucleic acid contained in the vector replicates, and the nucleic acid is thereby amplified. The host cell can be a eukaryotic cell or a prokaryotic cell. Suitable host cells include but are not limited to CHO cells, various COS cells, HeLa cells, HEK cells such as HEK 293 cells.

As used herein, a "vector" is a replicable nucleic acid. When the vector is transformed into an appropriate host cell, one or more heterologous proteins can be expressed from the vector. A vector includes those that are usually digested by restriction enzymes and ligated, into which the nucleic acid encoding the polypeptide or fragment thereof is introduced. The vector also includes those containing the nucleic acid encoding the polypeptide. A vector is used to introduce the nucleic acid encoding the polypeptide into a host cell, for amplifying the nucleic acid or for expressing/displaying the polypeptide encoded by the nucleic acid. A vector is generally kept free but can be designed to integrate the gene or part thereof into the chromosome of the genome. A vector of artificial chromosome is also considered, such as yeast artificial vector and mammalian artificial chromosome. The choice and use of such vector are well known to a person skilled in the art.

As used herein, a vector also includes a "virus vector" or a "viral vector". A viral vector is an engineered virus that is operably linked to a foreign gene to transfer (as a vehicle or shuttle) it into cell.

As used herein, an "expression vector" includes a vector capable of expressing DNA, said DNA being operably linked to a regulatory sequence such as a promoter region that can affect the expression of such DNA fragments. Such additional fragments may include promoter and terminator sequences, and can optionally include one or more origins of replication, one or more selective markers, enhancers, polyadenylation signals, etc. The expression vector is generally derived from plasmid or viral DNA or can contain both of the elements. Therefore, an expression vector refers to a recombinant DNA or RNA construct, such as plasmid, bacteriophage, recombinant virus, or other vectors, when introduced into appropriate host cells, resulting in the expression of the cloned DNA. Appropriate expression vectors are well known to a person skilled in the art and include those that can be replicated in eukaryotic and/or prokaryotic cells and those remain free or those are integrated into the host cell genome.

As used herein, "treating" a subject suffering from a disease or disease condition means that the subject's symptoms are partially or completely relieved or remain unchanged after treatment. Therefore, treatment includes prevention, treatment and/or cure. Prevention refers to preventing potential disease and/or preventing deterioration of symptoms or disease progression. Treatment also includes providing any pharmaceutical use of any antibody or antigen-binding fragment thereof and the composition provided herein.

As used herein, a "therapeutic effect" refers to the effect caused by a subject's treatment, the change, usually ameliorating or improving the symptoms of a disease or disease condition or curing the disease or disease condition.

As used herein, "therapeutically effective amount" or "therapeutically effective dose" refers to the amount of a substance, compound, material, or composition containing a compound which is at least sufficient to produce a therapeutic effect after administration to a subject. Therefore, it is the amount necessary to prevent, cure, improve, block or partially block the symptoms of a disease or disorder. Likewise, as used herein, a "prophylactically effective amount" or "prophylactically effective dose" refers to the amount of a substance, compound, material, or composition containing a compound, which when administered to a subject, will have the expected preventive effect, for example, preventing or delaying the occurrence or recurrence of a disease or symptom, reducing the possibility of occurrence or recurrence of a disease or symptom. A complete preventive effective dose does not have to occur by administering one dose and can only occur after a series of doses administered. Therefore, the prophylactically effective amount can be administered in one or more administrations.

As used herein, the term "subject" refers to mammal, such as human.

The antibody number used herein (such as B10, B11, H12) are only used to distinguish or identify antibodies or products and are not intended to indicate that such identification is a feature of the antibody or product according to the invention. A person skilled in the art will understand that, for example, for the purpose of differentiation or identification, other antibody or product may also use such identification, but it does not refer to the same or equivalent antibody or product. Similarly, the similar numbers or identifications used in the Examples are only for convenience of illustration. The antibody or product according to the invention is defined by the features described in the appended claims.

### Antibodies according to the invention

Provided is an antibody against PD-L1 (PD-L1 antibody) or an antigen-binding fragment thereof, wherein the antibody or the antigen-binding fragment thereof specifically recognizes and binds to PD-L1.

In an embodiment, the antibody or the antigen-binding fragment thereof is capable of specifically recognizing and binding to PD-L1, wherein the affinity (KD) is 1×10⁻⁹ M or less, preferably 5×10⁻¹⁰ M or less, more preferably 1×10⁻¹⁰ M or less.

In another embodiment, the antibody or the antigen-binding fragment thereof has at least one of the following:
1) blocking the interaction between PD-L1 and PD-1; 2) blocking the interaction between PD-L1 and CD80; 3) activating T cells; 4) inhibiting tumor growth.

In some embodiments, the antibody or the antigen-binding fragment thereof according to the invention are capable of specifically binding to PD-L1 (e.g. human PD-L1) and blocking its interaction with PD-1.

In some embodiments, the antibody or the antigen-binding fragment thereof according to the invention are capable of specifically binding to PD-L1 (e.g. human PD-L1) and blocking its interaction with CD80.

In some embodiments, the target tumors include but are not limited to those described as follows for the neoplastic disease. In some other embodiments, the antibody or the antigen-binding fragment thereof according to the invention can inhibit tumor growth by at least inhibiting 10%, preferably at least about 20%, more preferably at least about 30%, more preferably at least about 40%, more preferably at least about 50%, more preferably at least about 60%, more preferably at least about 70%, more preferably at least about 80%.

In an embodiment, the PD-L1 antibody comprises PD-L1 Antibody B10, PD-L1 Antibody B11, PD-L1 Antibody H12 or a combination thereof, particularly PD-L1 Antibody B11.

### Antibody B10

In an aspect, provided is Antibody B10 against PD-L1 or an antigen-binding fragment thereof, wherein the antibody comprises a light chain variable region and a heavy chain variable region, wherein
the heavy chain variable region comprises:
H CDR1, comprising an amino acid sequence as shown in SEQ ID NO:1,
H CDR2, comprising an amino acid sequence as shown in SEQ ID NO:2, and
H CDR3, comprising an amino acid sequence as shown in SEQ ID NO:3;
the light chain variable region comprises:
L CDR1, comprising an amino acid sequence as shown in SEQ ID NO:5,
L CDR2, comprising an amino acid sequence as shown in SEQ ID NO:6, and
L CDR3, comprising an amino acid sequence as shown in SEQ ID NO:7.

In another embodiment, the PD-L1 Antibody B10 comprises a heavy chain variable region (VH),
wherein the heavy chain variable region comprises an amino acid sequence as shown in SEQ ID NO:4 or an amino acid sequence having at least 85%, at least 90%, at least 95% or more sequence identity to SEQ ID NO:4.

In another embodiment, the PD-L1 Antibody B10 comprises a light chain variable region (VL),
wherein the light chain variable region comprises an amino acid sequence as shown in SEQ ID NO:8 or an amino acid sequence having at least 85%, at least 90%, at least 95% or more sequence identity to SEQ ID NO:8.

In yet another embodiment, the PD-L1 Antibody B10 comprises a heavy chain constant region (CH),
wherein the heavy chain constant region comprises an amino acid sequence as shown in SEQ ID NO:26 or an amino acid sequence having at least 85%, at least 90%, at least 95% or more sequence identity to SEQ ID NO:26.

In yet another embodiment, the PD-L1 Antibody B10 comprises a light chain constant region (CL),
wherein the light chain constant region comprises an amino acid sequence as shown in SEQ ID NO:25 or an amino acid sequence having at least 85%, at least 90%, at least 95% or more sequence identity to SEQ ID NO:25.

### Antibody B11

In another aspect, provided is Antibody B11 against PD-L1 or an antigen-binding fragment thereof,
wherein the antibody comprises a light chain variable region and a heavy chain variable
region, wherein
the heavy chain variable region comprises:
H CDR1, comprising an amino acid sequence as shown in SEQ ID NO:9,
H CDR2, comprising an amino acid sequence as shown in SEQ ID NO:10, and
H CDR3, comprising an amino acid sequence as shown in SEQ ID NO:11;
the light chain variable region comprises:
L CDR1, comprising an amino acid sequence as shown in SEQ ID NO:13,
L CDR2, comprising an amino acid sequence as shown in SEQ ID NO:14, and
L CDR3, comprising an amino acid sequence as shown in SEQ ID NO:15.

In an embodiment, the PD-L1 Antibody B11 comprises a heavy chain variable region (VH),
wherein the heavy chain variable region comprises an amino acid sequence as shown in SEQ ID NO:12 or an amino acid sequence having at least 85%, at least 90%, at least 95% or more sequence identity to SEQ ID NO:12.

In an embodiment, the PD-L1 Antibody B11 comprises a light chain variable region (VL),
wherein the light chain variable region comprises an amino acid sequence as shown in SEQ ID NO:16 or an amino acid sequence having at least 85%, at least 90%, at least 95% or more sequence identity to SEQ ID NO:16.

In a preferable embodiment, the PD-L1 Antibody B11 comprises a heavy chain variable region (VH) and a light chain variable region (VL),
wherein the heavy chain variable region comprises an amino acid sequence as shown in SEQ ID NO:12; the light chain variable region comprises an amino acid sequence as shown in SEQ ID NO:16

In yet another embodiment, the PD-L1 Antibody B11 comprises a heavy chain constant region (CH),
wherein the heavy chain constant region comprises an amino acid sequence as shown in SEQ ID NO:26 or an amino acid sequence having at least 85%, at least 90%, at least 95% or more sequence identity to SEQ ID NO:26.

In yet another embodiment, the PD-L1 Antibody B11 comprises a light chain constant region (CL),
wherein the light chain constant region comprises an amino acid sequence as shown in SEQ ID NO:25 or an amino acid sequence having at least 85%, at least 90%, at least 95% or more sequence identity to SEQ ID NO:25.

In another specific embodiment, the PD-L1 Antibody B11 comprises a heavy chain,
wherein the heavy chain comprises an amino acid sequence as shown in SEQ ID NO:28 or an amino acid sequence having at least 85%, at least 90%, at least 95% or more sequence identity to SEQ ID NO:28.

In another specific embodiment, the PD-L1 Antibody B11 comprises a light chain,
wherein the light chain comprises an amino acid sequence as shown in SEQ ID NO:27 or an amino acid sequence having at least 85%, at least 90%, at least 95% or more sequence identity to SEQ ID NO:27.

In a particularly preferable embodiment, the PD-L1 Antibody B11 comprises a heavy chain and a light chain,
wherein the heavy chain comprises an amino acid sequence as shown in SEQ ID NO:28, and the light chain comprises an amino acid sequence as shown in SEQ ID NO:27.

### Antibody H12

In another aspect, provided is Antibody H12 against PD-L1 or an antigen-binding fragment thereof,
wherein the antibody comprises a light chain variable region and a heavy chain variable
region, wherein
the heavy chain variable region comprises:
H CDR1, comprising an amino acid sequence as shown in SEQ ID NO:17,
H CDR2, comprising an amino acid sequence as shown in SEQ ID NO:18, and
H CDR3, comprising an amino acid sequence as shown in SEQ ID NO:19;
the light chain variable region comprises:
L CDR1, comprising an amino acid sequence as shown in SEQ ID NO:21,
L CDR2, comprising an amino acid sequence as shown in SEQ ID NO:22, and
L CDR3, comprising an amino acid sequence as shown in SEQ ID NO:23.

In another embodiment, the PD-L1 Antibody H12 comprises a heavy chain variable region,
wherein the heavy chain variable region comprises an amino acid sequence as shown in SEQ ID NO:20 or an amino acid sequence having at least 85%, at least 90%, at least 95% or more sequence identity to SEQ ID NO:20.

In another embodiment, the PD-L1 Antibody H12 comprises a light chain variable region,
wherein the light chain variable region comprises an amino acid sequence as shown in SEQ ID NO:24 or an amino acid sequence having at least 85%, at least 90%, at least 95% or more sequence identity to SEQ ID NO:24.

In yet another embodiment, the PD-L1 Antibody H12 comprises a heavy chain constant region (CH),
wherein the heavy chain constant region comprises an amino acid sequence as shown in SEQ ID NO:26 or an amino acid sequence having at least 85%, at least 90%, at least 95% or more sequence identity to SEQ ID NO:26.

In yet another embodiment, the PD-L1 Antibody H12 comprises a light chain constant region (CL),
wherein the light chain constant region comprises an amino acid sequence as shown in SEQ ID NO:25 or an amino acid sequence having at least 85%, at least 90%, at least 95% or more sequence identity to SEQ ID NO:25.

### Neoplastic diseases

The blocking of PD-L1 by the antibody according to the invention can enhance the immune response to cancer cells in patients. PD-L1 is abundant in a variety of human cancers. The interaction between PD-1 and PD-L1 leads to decrease in tumor-infiltrating lymphocytes, decrease in T cell receptor-mediated proliferation, and immune escape of cancer cells. Inhibiting the interaction between PD-L1 and PD-1 can reverse immunosuppression.

The antibody or antigen-binding fragment thereof according to the invention can be used to treat a neoplastic disease. The preferable neoplastic disease (or cancer) which can be prevented and/or treated with the antibody or antigen-binding fragment thereof according to the invention comprises cancers that generally respond to immunotherapy. Non-limiting examples of treatable cancers include but are not limited to lung cancer, ovarian cancer, colon cancer, rectal cancer, melanoma, renal cancer, bladder cancer, breast cancer, liver cancer, lymphoma, malignant hematological disease, head and neck cancer, glioma, stomach cancer, nasopharyngeal cancer, laryngeal cancer, cervical cancer, uterine corpus tumor and osteosarcoma. Examples of other cancers include, but are not limited to, bone cancer, pancreatic cancer, skin cancer, prostate cancer, skin or intraocular malignant melanoma, uterine cancer, anal cancer, testicular cancer, fallopian tube cancer, endometrial cancer, vagina cancer, vulva cancer , Hodgkin's disease, non-Hodgkin's lymphoma, esophageal cancer, small intestine cancer, endocrine system cancer, thyroid cancer, parathyroid cancer, adrenal cancer, soft tissue sarcoma, urethral cancer, penile cancer, chronic or acute leukemia, including acute myeloid leukemia, chronic myeloid leukemia, acute lymphoblastic leukemia, chronic lymphocytic leukemia, childhood solid tumor, lymphatic lymphoma, bladder cancer, renal or ureteral cancer, renal pelvis cancer, central nervous system (CNS) tumor, primary CNS lymphoma, tumor angiogenesis, spinal tumor, brainstem nerve glioma, pituitary adenoma, Kaposi sarcoma, epidermal carcinoma, squamous cell carcinoma, T cell lymphoma, environmentally-induced cancer, including asbestos-induced cancers, and combinations of said cancers. The antibody according to the invention can also be used to treat metastatic cancers, particularly metastatic cancers that express PD-L1. In yet another embodiment, the neoplastic disease (or cancer) is lung cancer, ovarian cancer, colorectal cancer, melanoma, renal cancer, bladder cancer, breast cancer, liver cancer, lymphoma, malignant hematological disease, head and neck cancer, glioma, stomach cancer, nasopharyngeal cancer, laryngeal cancer, cervical cancer, uterine corpus carcinoma or osteosarcoma, particularly colorectal cancer or non-small cell lung cancer. In an embodiment, the neoplastic disease is a PD-L1-positive tumor.

In a preferable embodiment, the antibody comprises PD-L1 Antibody B10, PD-L1 Antibody B11, PD-L1 Antibody H12 or a combination thereof, particularly PD-L1 Antibody B11.

### Nucleic acids, vectors and antibody production process

In another aspect, provided is an isolated nucleic acid molecule, encoding the antibody or the antigen-binding fragment thereof according to the invention as described above. In some embodiments, the nucleotide sequence of the nucleic acid molecule can be codon-optimized for the host cell used for expression. Provided is also an expression vector, comprising at least one of the nucleic acid molecules according to the invention as described above.

In an aspect, provided is a host cell, which is transformed with at least one of the nucleic acid molecules or the expression vectors according to the invention as described above.

In another aspect, provided is a process for preparing an antibody against PD-L1 or an antigen-binding fragment thereof, comprising,
(i) culturing the host cell according to the invention under a condition suitable for the expression of a nucleic acid molecule or an expression vector, and (ii) isolating and purifying the antibody or the antigen-binding fragment thereof expressed by the nucleic acid molecule or the expression vector.

### Pharmaceutical compositions

Provided is also a pharmaceutical composition, comprising the antibody according to the invention, and a pharmaceutically acceptable carrier. In an embodiment, the above antibody comprises PD-L1 Antibody B10, PD-L1 Antibody B11, PD-L1 Antibody H12 or a combination thereof, particularly PD-L1 Antibody B11.

As used herein, the "pharmaceutically acceptable carrier" includes any and all physiologically compatible solvents, dispersing media, coatings, antibacterial and antifungal agents, isotonic and absorption delaying agents, or the like. Preferably, the carrier is suitable for intravenous, intramuscular, subcutaneous, parenteral, spinal or epidermal administration (such as by injection or infusion). According to the route of administration, the active compound (i.e. the antibody molecule) and the immunoconjugate can be encapsulated in some material to protect the compound from acids and other natural conditions that can inactivate the compound.

The pharmaceutical composition according to the invention may also contain a pharmaceutically acceptable antioxidant. Examples of pharmaceutically acceptable antioxidants include: (1) water-soluble antioxidants, such as ascorbic acid, cysteine hydrochloride, sodium bisulfate, sodium metabisulfite, sodium sulfite, or the like; (2) oil-soluble antioxidants, such as ascorbyl palmitate, butylated hydroxyanisole (BHA), butylated hydroxytoluene (BHT), lecithin, propyl gallate, α-tocopherol, or the like; and (3) metal chelating agents, such as citric acid, ethylenediaminetetraacetic acid (EDTA), sorbitol, tartaric acid, phosphoric acid, or the like.

The composition may also contain an adjuvant, such as preservative, wetting agent, emulsifying agent and dispersing agent.

The prevention of microorganisms can be ensured by sterilization procedures or by incorporating various antibacterial and antifungal agents such as parabens, chlorobutanol, phenol, sorbic acid, or the like. In many cases, the composition preferably includes an isotonic agent, for example, sugar, polyol such as mannitol, sorbitol or sodium oxide. By incorporating an absorption-delaying agent in the composition, such as monostearate and gelatin, the prolonged absorption of injectable drugs can be achieved.

The pharmaceutically acceptable carrier includes sterile aqueous solution or dispersion and powder for the temporary preparation of sterile injection or dispersion. Use of these media and reagents for pharmaceutically active substance is well known in the art. Conventional media or reagents, except for any range incompatible with the active compound, may be in the pharmaceutical composition according to the invention. A supplementary active compound can also be incorporated into the composition.

A therapeutic composition must generally be sterile and stable under the conditions of preparation and storage. The composition can be formulated into a solution, microemulsion, liposome or other ordered structure suitable for high drug concentration. The carrier can be a solvent or dispersant containing, for example, water, ethanol, polyol (e.g., glycerol, propylene glycol, and liquid polyethylene glycol, or the like) and a suitable mixture thereof. For example, by using a coating, such as lecithin, by maintaining the desired particle size in the case of a dispersion, and by using a surfactant, proper fluidity can be maintained.

By mixing the active compound in a suitable solvent in a desirable amount and adding one or a combination of the above-listed ingredients as needed, followed by sterile microfiltration, a sterile injection can be prepared. Generally, a dispersion is prepared by incorporating the active compound into a sterile carrier containing a basic dispersing medium and other required ingredients listed above. For sterile powder for the preparation of a sterile injection, a preferable preparation process is vacuum drying and freeze-drying (lyophilization), and the powder of the active ingredient plus any additional required ingredients are obtained from the pre-sterile filtered solution.

The amount of the active ingredient that can be combined with the carrier material to prepare a single dosage form varies according to the subject to be treated and the specific mode of administration. The amount of active ingredient that can be combined with a carrier material to prepare a single dosage form is generally the amount of the composition that produces a therapeutic effect. Generally, based on 100%, this amount ranges from about 0.01% to about 99% of the active ingredient, preferably about 0.1% to about 70%, and most preferably about 1% to about 30% of the active ingredient, combined with a pharmaceutically acceptable carrier.

The dosage regimen can be adjusted to provide the optimal desired response (e.g., therapeutic response). For example, a single bolus can be administered, several divided doses can be administered over time, or according to the urgency of the conditions to be treated, the dose can be reduced or increased proportionally. It is particularly advantageous to formulate a parenteral composition into unit dosage form for easy administration and uniform dosage. The unit dosage form used herein refers to a physically discrete unit suitable as a unit dose for the subject to be treated; each unit contains a predetermined amount of active compound, and the predetermined amount is calculated in combination with the required pharmaceutical carrier to produce a desired therapeutic effect. The specific description of the unit dosage form of the invention is limited and directly depends on (a) the unique characteristics of the active compound and the specific therapeutic effect to be achieved, and (b) inherent limitations in the art for formulating such active compounds for sensitivity of an individual to be treated.

For the administration of antibody molecules, the dose range is about 0.0001 to 100 mg/kg, more often 0.01 to 20 mg/kg of the patient's body weight. For example, the dose can be 0.3 mg/kg body weight, 1 mg/kg body weight, 3 mg/kg Body weight, 5 mg/kg body weight, 10 mg/kg body weight or 20 mg/kg body weight, or within the range of 1-20 mg/kg. An exemplary treatment regimen requires administration once a week, once every two weeks, once every three weeks, once every four weeks, once a month, once every 3 months, once every 3-6 months, or a slightly short initial dosing interval and a long interval of late dosing. In an embodiment, the dose used can be 1200 mg administered every three weeks. The mode of administration can be intravenous drip.

Alternatively, the tumor-targeted antibody molecule can also be administered as a sustained release formulation, in which a less frequent administration is required. The dosage and frequency vary according to the half-life of the antibody molecule in the patient. Generally, a human antibody shows the longest half-life, followed by a humanized antibody, a chimeric antibody, and a non-human antibody. The dosage and frequency of administration differ depending on whether the treatment is prophylactic or therapeutic. In a prophylactic application, relatively low doses are given at less frequent intervals over a long period of time. Some patients continue to receive treatment for the rest of their lives. In a therapeutic application, it is sometimes necessary to administer higher doses at shorter intervals until the progression of the disease is reduced or stopped, preferably until the patient shows partial or complete improvement of the symptoms of the disease. Afterwards, the patient can be administered in a prophylactic regimen.

The actual dosage level of the active ingredient in the pharmaceutical composition may vary to obtain an amount of the active ingredient that can effectively achieve the desired therapeutic response to the specific patient, composition and administration mode without being toxic to the patient. The selected dosage level depends on various pharmacokinetic factors, including the activity of the specific composition of the invention used, the route of administration, the time of administration, the excretion rate of the specific compound used, the duration of treatment, and the specific application of the composition with other drugs, compounds and/or materials, the age, gender, weight, condition, general health and medical history of the patient being treated, and similar factors known in the medical field.

An "effective amount" of the antibody or antigen-binding fragment thereof according to the invention preferably results in a decrease in the severity of symptoms, an increase in the frequency and duration of the asymptomatic period of the disease, or prevention of injury or disability caused by the pain of the disease. For example, for the treatment of a tumor, an "effective amount" of the antibody or antigen-binding fragment thereof according to the invention preferably inhibits cell growth or tumor growth by at least about 10%, preferably at least about 20 %, more preferably at least about 30%, more preferably at least about 40%, more preferably at least about 50%, more preferably at least about 60%, more preferably at least about 70%, more preferably at least about 80%, in comparison to an untreated subject. The ability to inhibit tumor growth can be evaluated in an animal model system that predicts the efficacy to human tumors. Alternatively, it can also be evaluated by checking the ability to inhibit cell growth, and this inhibition can be determined *in vitro* by a test well known to a person skilled in the art. An effective amount of the antibody or antigen-binding fragment thereof according to the invention can reduce tumor size, or relieve symptoms of the subject in other ways, such as preventing and/or treating metastasis or recurrence. A person skilled in the art can determine this amount based on the following factors, for example, the size of the subject, the severity of the subject's symptoms and the particular composition or route of administration selected.

The antibody or antigen-binding fragment thereof according to the invention or the pharmaceutical composition according to the invention can be administered through one or more administration routes using one or more methods known in the art. It should be understood by a person skilled in the art that the route of administration and/or mode varies according to the desired results. The preferable route of administration for the antibody according to the invention includes intravenous, intramuscular, intradermal, intraperitoneal, subcutaneous, spinal or other parenteral routes of administration, such as injection or infusion. The phrase "parenteral administration" as used herein refers to a mode of administration other than enteral and local administration, usually by injection, including but not limited to intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion.

Alternatively, the antibody or antigen-binding fragment thereof according to the invention or the pharmaceutical composition according to the invention can also be administered by a non-parenteral route, such as topical, epidermal or mucosal route, for example, intranasal, oral, vaginal, rectal, sublingual or local.

The active compound can be prepared with a carrier that protects the compound from being quickly released, such as a controlled release formulation, including implant, transdermal patch and microencapsulated delivery system. A biodegradable, biocompatible polymer can be used, such as ethylene vinyl acetate, polyanhydrides, polyglycolic acid, collagen, polyorthoester and polylactic acid. Many processes for preparing such preparations are protected by patents or generally known to a person skilled in the art.

The antibody or antigen-binding fragment thereof according to the invention in the pharmaceutical composition can also be conjugated with a therapeutic moiety such as cytotoxin, radioisotope, biologically active protein or the like.

Cytotoxin includes any agent that is harmful to cells (e.g., killing cells). Examples include, but are not limited to: paclitaxel, cytochalasin B, gramicidin D, ethidium bromide, emetine, mitomycin, epipodophyllotoxin glucopyranoside, epipodophyllotoxin thiophene glycoside, vincristine, vinblastine, colchicine, adriamycin, daunorubicin, dihydroxyanthracenedione, mitoxantrone, plicamycin, actinomycin D, 1-dehydrotestosterone, glucocorticoid, procaine, tetracaine, lidocaine, propranolol and puromycin and their analogs or homologs.

Therapeutic agents that can be used for conjugation also include, for example: antimetabolite (e.g., methotrexate, 6-mercaptopurine, 6-thioguanine, cytarabine, 5-fluorouracil, dacarbazine), alkylating agent (e.g. nitrogen mustard, chlorambucil, phenylalanine mustard, carmustine (BSNU) and lomustine (CCNU), cyclophosphamide, busulfan, dibromomannitol, streptozotocin, mitomycin C and cis-dichlorodiamine platinum (II) (DDP) cisplatin), anthramycins (e.g., daunorubicin (previously known as daunomycin) and adriamycin), antibiotics (e.g., Actinomycin D (previously known as Actinomycin), bleomycin, plicamycin and anthramycin (AMC)), and antimitotic agent (e.g., vincristine and vinblastine).

Other preferable examples of therapeutic cytotoxins that can be conjugated to the antibody according to the invention include duocarmycin, calicheamicin, maytansine, auristatin, and their derivatives.

The cytotoxin can be conjugated to the antibody according to the invention using linker technology in the art. Examples of linker types that have been used to conjugate cytotoxin to antibody include but are not limited to hydrazone, thioether, ester, disulfide, and peptide-containing linker. For example, a linker that is easily cleaved by low pH or protease in the lysosomal compartment may be selected, and the protease is, for example, a protease that is preferably expressed in tumor tissues, such as cathepsin (e.g., cathepsin B, C, D).

The antibody according to the invention can also be conjugated with a radioisotope to produce a cytotoxic radiopharmaceutical, which is also called a radioactive antibody conjugate. Examples of radioisotopes that can be conjugated to an antibody for diagnostic or therapeutic use include, but are not limited to, Iodine 131, Indium 111, Yttrium 90, and Lutetium 177. Processes for preparing radioactive antibody conjugates are well known in the art.

The antibody according to the invention can also be conjugated with a protein having required biological activity and can be used to modify a specific biological response. Such biologically active proteins include, for example: toxin with enzymatic activity or active fragment thereof, such as abrin, ricin A, pseudomonas exotoxin or diphtheria toxin; protein, such as tumor necrosis factor or interferon-y; or biological response modifier, such as lymphokines, interleukin-1 (""IL-1"), interleukin-2 ("IL-2"), interleukin-6 ("IL-6"), interleukin-10 ("IL-10"), granulocyte macrophage colony stimulating factor ("GM-CSF"), granulocyte colony stimulating factor ("G-CSF") or other immune factors such as IFN, etc.

### Combined therapies

The antibody or pharmaceutical composition according to the invention can be administered in combination with a chemotherapeutic agent or antibody targeting other tumor antigen. The antibody according to the invention and the chemotherapeutic agent or antibody targeting other tumor antigen can be administered at once or separately. When administered separately (when different administration schedules are used), they can be administered continuously without interruption or at predetermined intervals. The antibody according to the invention or the pharmaceutical composition according to the invention can also be combined with radiotherapy, for example, including the administration of ionizing radiation to the patient, which is earlier than, during the process of, and/or later than the administration process of the antibody or pharmaceutical composition according to the invention.

### Kits

A kit is also encompassed within the scope of the invention, which comprises the antibody or the antigen-binding fragment thereof according to the invention, and instructions for use. The kit generally includes a label indicating the intended use and/or method of use of the contents of the kit. The term label includes any written or recorded materials provided on or with the kit or otherwise provided with the kit.

### Beneficial effects

The antibody according to the invention has very strong affinity for the target antigen, activates T cells, can exhibit excellent *(in vivo)* anti-tumor activity, excellent stability, lower side effects, and the effect is better than the prior art.

### Examples

Further understanding of the invention can be obtained by referring to some specific examples provided herein, and these examples are only used to illustrate the present invention rather than any limitation to the scope thereof. It is obvious that various changes and modifications can be made to the invention without departing from the spirit thereof, and therefore these changes and modifications are also within the claimed protection scope of the present application. Unless otherwise stated, the ratios including percentages used herein are calculated on a weight basis.

The reagents and instruments used in the Examples are all commercially available. Tecentriq can be obtained from Roche.

### Example 1 Antibody screening

Peripheral blood of 106 healthy adults (53 males and 53 females) were collected, and PBMC (peripheral blood mononuclear cell) cells were separated using lymphocyte separation solution, and a total of 2×10⁹ cells were collected. Total RNAs were extracted with Trizol method and were reverse transcribed into cDNA. By referring to the method in "Recombinant Antibody" (Science Press, 2005), the conventional PCR method was used to amplify the variable region genes of different antibody subtypes, and the antibody variable region genes were cloned into the pDF vector digested with the same enzyme by conventional molecular biology techniques. *E. coli* XL1-Blue (Agilent Technology) was transformed by electroporation. After expansion with SB medium, 1×10¹³ pfu helper virus VCSM13 (BioVector NTCC Inc.) was added for infection to obtain a primary phage antibody library, and the primary antibody library and BS1365 bacteria were mixed in proportion (MOI of infection> 200) to construct a large capacity antibody library.

After blocking the immunoassay tube (Maxisorp immunoassay tube, Thermo Nunc) coated with recombinant PD-L1 (product of R&D company) with 5% skimmed milk powder, the above phage antibody library was added and incubated at 37°C for 2 h; unbound phages were discarded, TBS solution was used for washing 5 times with non-specifically adsorbed phages being completely washed away; 1 mL of elution buffer (0.1 mol/L glycine-HCl, pH 2.2) was added to elute the phages and 40 µL of 2 mol/L Tris solution was used for neutralization. XL1-Blue bacteria (Agilent Technology) at logarithmic phase, SB medium (SB medium: tryptone 30 g, yeast extract 20 g, MOPS 10 g, dissolved in 950 mL deionized water, pH adjusted to 7.0 with sodium hydroxide, volume metered to 1 L, autoclaved) and the helper phage VCSM13 were added for amplification and enrichment. The process was repeated for 3-4 times, and the eluted phages were used to infect the freshly prepared XL1-Blue bacteria (logarithmic phase)-coated culture plate. After incubation overnight at 37°C, a single clone was obtained (for specific methods and reagents, reference can be made to: "Phage Display", Humana Press).

Recombinant PD-L1 was incubated with a 96-well plate at 4°C for 16 h, and then the non-specific binding was blocked with BSA solution. From the phage library with obvious enrichment after screening, about 1000 (12 96-well plates)) of single phage clones were selected and cultured in 500 µL of 2YT liquid medium supplemented with carbenicillin and ampicillin for 24 h, incubated with the recombinant PD-L1 solution for 2 h. The mixture was added to the 96-well plate coupled with the above-mentioned recombinant PD-L1 and incubated for 15 min. After washing with PBST, incubation with HRP-conjugated anti-phage antibody (anti-M13 HRP) was performed, and finally a substrate was added for color development to determine the amount of phage bound to the 96-well plate. As the PD-L1 in the solution bound tightly to the phages with higher affinity, it competitively inhibited binding of the phages to the PD-L1 coupled to the 96-well plate, and thus there was lower OD reading in ELISA. Using this experiment, 12 candidate antibodies with relatively high affinity and specific sequences were initially selected for further testing and screening.

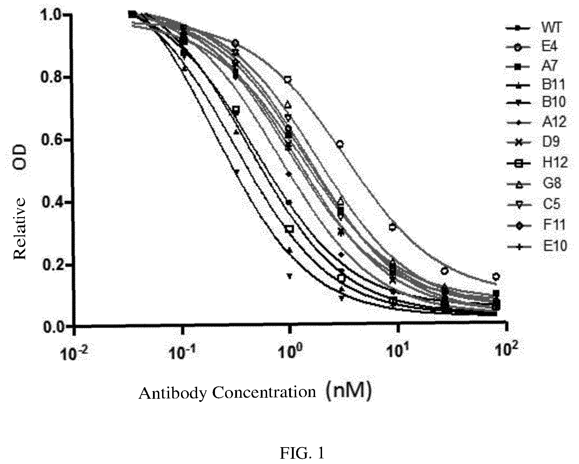

The recombinant PD-L1 was incubated with a 96-well plate at 4°C for 16 h, and non-specific binding was blocked with BSA solution. The phage clones selected from the above competitive ELISA were incubated with recombinant PD-L1 solutions of different concentrations (0.03 nM-90 nM) for 2 h, and the mixture was added to the 96-well plate coupled with the above-mentioned recombinant PD-L1 and incubated for 15 min. After washing with PBST, incubation with HRP-conjugated anti-phage antibody (anti-M13 HRP) was performed, and finally a substrate was added for color development to determine the amount of phages bound to the 96-well plate. GraphPad Prism was used to make binding curve based on the OD reading in ELISA. The results were shown in Fig. 1 (WT is the positive control, an antibody having the same sequence as Tecentriq).

Sequence analysis was performed for the 3 candidate antibodies with the highest affinity (_{HZPDL1-}B10, _{HZPDL1-}B11, _{HZPDL1-}H12 (in the following text and the drawings, the respective abbreviations B10, B11, H12 were used for convenience)).

The sequences are as follows:
B10 clone has HCDR1 with the sequence of SEQ ID NO:1, HCDR2 with the sequence of SEQ ID NO:2, HCDR3 with the sequence of SEQ ID NO:3, VH with the sequence of SEQ ID NO:4; LCDR1 with the sequence of SEQ ID NO:5, LCDR2 with the sequence of SEQ ID NO:6, L CDR3 with the sequence of SEQ ID NO:7, VL with the sequence of SEQ ID NO:8.
B11 clone has HCDR1 with the sequence of SEQ ID NO:9, HCDR2 with the sequence of SEQ ID NO:10, HCDR3 with the sequence of SEQ ID NO:11, VH with the sequence of SEQ ID NO:12; LCDR1 with the sequence of SEQ ID NO:13, L CDR2 with the sequence of SEQ ID NO:14, L CDR3 with the sequence of SEQ ID NO:15, VL with the sequence of 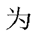 SEQ ID NO:16.
H12 clone has HCDR1 with the sequence of SEQ ID NO:17, HCDR2 with the sequence of SEQ ID NO:18, HCDR3 with the sequence of SEQ ID NO:19, VH with the sequence of SEQ ID NO:20; LCDR1 with the sequence of SEQ ID NO:21, L CDR2 with the sequence of SEQ ID NO:22, L CDR3 with the sequence of SEQ ID NO:23, VL with the sequence of SEQ ID NO:24.

### Example 2 Antibody expression

The obtained variable region genes of B10, B11, H12 clones were fused with human IgG constant region gene (the light chain constant region sequence is SEQ ID NO:25; the heavy chain constant region sequence is SEQ ID NO:26) and cloned into the expression vector pCDNA3.4 (Thermo Fisher). The obtained eukaryotic expression vectors were transiently transfected into Expi-CHO cells (Thermo Fisher). After about 8 days of serum-free cell culture, the culture supernatant was harvested and subjected to ELISA test (using goat anti-human IgG and horseradish enzyme-labeled goat anti-human IgG for double-sandwich ELISA assay to detect the content of the antibody in the supernatant). The antibody expression level in the serum was determined, and the culture supernatant containing the candidate antibody was purified with protein A resin to obtain the target antibody.

### Example 3 Analyzing the specific recognition activity of the antibody

In this Example, ELISA assay was used to determine binding of the candidate antibody to human, monkey, and mouse PD-L1.

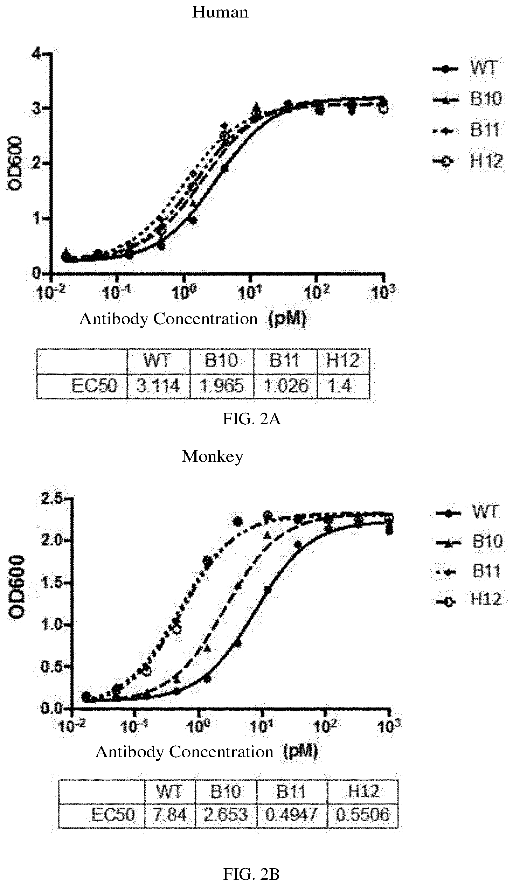

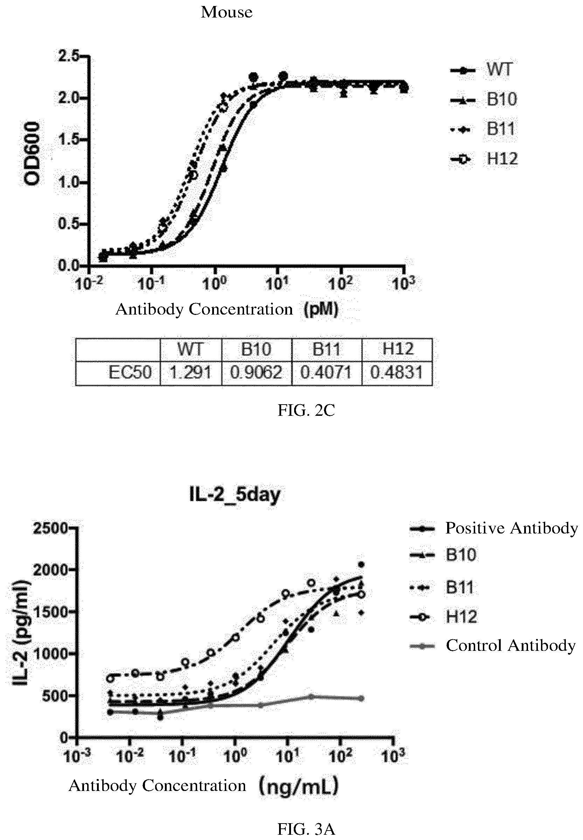

Human, monkey, and mouse PD-L1 (R&D) were diluted with PBS to 5 µg/ml and to coat 96-well plates. After incubation overnight at 4°C, the coating solution was discarded the next day and washed with PBST. 200 µl of BSA blocking solution was added into each well and incubated for 1 h at room temperature. Washing was performed three times with PBST, and the candidate antibodies and the positive control antibody (WT, positive control, an antibody with the same sequence as Tecentriq) were 2-fold serial diluted to make 11 concentrations with 1 nM as the highest concentration. Then 100 µl of antibody was added into each well and incubated for 2 h at room temperature. Washing was performed three times with PBST, and anti-human Fc-HRP antibody (Sino Biological) was added and incubated for 1 h at room temperature. Finally, each well was washed four times with PBST, TMB color developing solution (Beijing Tiangen BioTech) was added, and the color was developed at room temperature. After the color development was stopped, the OD value was determined with a microplate reader at 600 nm. The ELISA results were shown in Fig. 2 (A, B, and C are human, monkey, and mouse PD-L1, respectively, and the EC₅₀ unit is pM). Candidate Antibody B10, B11 and H12 can bind to human, monkey and mouse PD-L1 proteins.

### Example 4 Antibody affinity analysis

In this Example, BIAcore is used to determine antibody affinity. In this experiment, the human antibody capture kit (GE, BR100839) was used to couple the anti-human IgG antibody to the S series CM5 sensor chip (GE, BR100530) through amino before the ligand and analyte binding. Recombinant anti-PD-Ll antibodies, B10, B11, and H12 were diluted with HBS-EP+Buffer as ligands. The recombinant His-tagged PD-L1 was diluted with HBS-EP+Buffer to 8 µg/ml, 4 µg/ml, 1.6 µg/ml, 0.64 µg/ml, 0.256 µg/ml and 0.102 µg/ml as analytes. In Biacore Wizard mode, affinity analysis experiments were performed in a multi-cycle manner. The test of each sample included 3 Start up, 1 zero concentration control, 6 gradient concentration samples and 1 repeated concentration sample. After each cycle, 10 mM Glycine HCl PH2.1 regeneration solution is used to regenerate the chip. Each concentration cycle of the analyte was set as follows: capture time 180s, ligand solution flow rate 10 µl/min, ligand and analyte binding time 180 s, analyte solution flow rate 30 µl/min; dissociation time 2400 s. Raw data was introduced into the analysis software, the zero concentration control was subtracted, and the reference channel was subtracted to eliminate the volume effect. The 1:1 binding mode in the Kinetics analysis method was used to fit the graph and the affinity data of the antigen antibody was calculated by regression analysis using the analysis software based on the binding curve. The experimental results showed that the candidate antibodies B10, B11 and H12 can recognize the target antigen with high affinity (Table 1).

**Table 1**

| Antibody clone | Ka (1/Ms) | Kd (1/s) | KD (M) |
|---|---|---|---|
| B10 | 1.435×10⁶ | 2.359×10⁻³ | 1.644×10⁻⁹ |
| B11 | 1.361×10⁶ | 8.567×10⁻⁴ | 6.294×10⁻¹⁰ |
| H12 | 1.190×10⁶ | 2.609×10⁻³ | 2.194×10⁻⁹ |

### Example 5 T cell activation

In this example, the mixed lymphocyte reaction experiment (MLR) was used to test the T cell activation ability of the candidate PD-L1 antibodies. Human Peripheral Blood Lymphocyte Separation Solution (Ficoll) was used to isolate PBMC from heparinized healthy fresh human blood, and then Human Monocyte Isolation Kit II (Miltenyi) was used to isolate monocytes from PBMC. Monocytes were plated into a 96-well plate at a density of 2.5×10⁴ cells per well, and 25 ng/ml of GM-CSF (Sino Biological) and 50 ng/ml of IL-4 (Sino Biological) were added for culture at 37°C with the fresh medium replaced every 3 days. After 7 days of culture, the monocytes were differentiated into dendritic cells. On the 7^{th} day of dendritic cell differentiation and maturation, CD4+ T cell isolation kit (Miltenyi) was used to isolate CD4+ T cells from PBMC of another healthy person. Then 2×10⁵/well of CD4+ T cells and dendritic cells were co-cultured in RPMI medium (Invitrogen) containing different concentrations of antibody. After 5 days, IPNγ ELISA kit (R&D) and IL-2 ELISA kit (eBioscience) were used to determine the contents of IPNγ and IL-2 secreted by activated T cells in the culture supernatant.

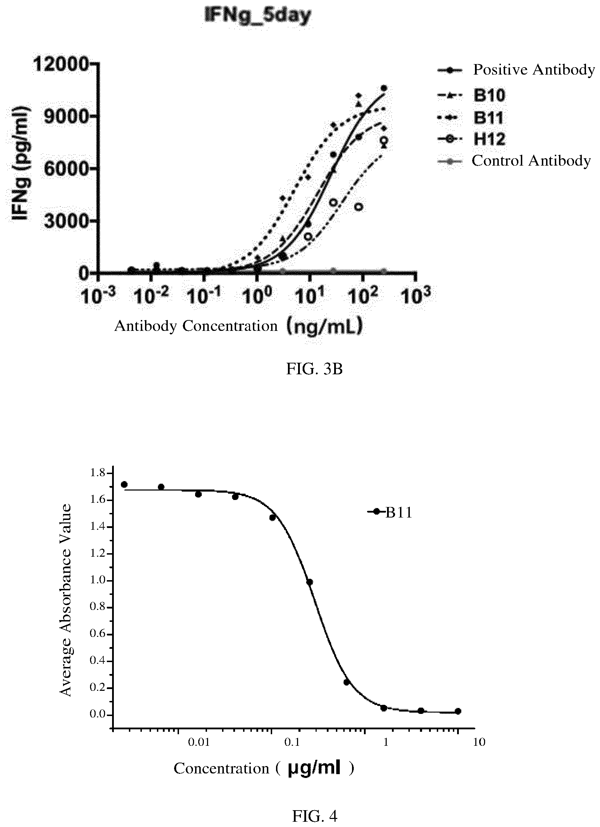

The results were shown in Fig. 3 (the positive antibody is an antibody having the same sequence as Tecentriq, and the control antibody is an isotype IgG control, human IgG1). Antibodies B10, B11 and H12 can activate T cells and increase the expression levels of IFNγ and IL-2.

### Example 6 The antibody blocks the interaction between PD-1 and PD-L1

B11 clone variable region gene and human IgG constant region gene (the light chain constant region sequence is SEQ ID NO: 25; the heavy chain constant region sequence is SEQ ID NO: 26) were fused and expressed to obtain a full antibody molecule, wherein the light chain amino acid sequence is SEQ ID NO: 27; the heavy chain amino acid sequence is SEQ ID NO: 28), and the biological activity was analyzed.

The activity of antibody to block the interaction between PD-1 and PD-L1 was analyzed. hPD-L1-Fc protein (R&D) was diluted in coating buffer to a coating concentration of 1 µg/mL, then added at 100 µl/well to the wells of the coating plate, and coating was performed overnight at 4°C. The plate was washed three times with washing solution (PBST), added with 2% of bovine serum albumin in the corresponding well at 100 µL/well, and blocked for 2 h at 37°C. The highest concentration of B11 was 10 µg/mL, and diluted at 2.5 folds, with a total of 10 concentrations. Biotin-labeled PD-1 (hPD-1-Fc-Biotin) (R&D) was diluted with diluent (1% BSA) to 0.25 µg/mL and the solution was mixed with the gradient-diluted B11 antibody at 1:1. The mixture was added to the blocked ELISA reaction wells, 180 µL/well, and triplicate wells were set for each sample concentration. The plate was covered with sealing membrane and placed horizontally at 37°C for 2 h. The plate was washed three times with the washing solution, added with HRP-labeled avidin (Shanghai Beyotime Biotech), and placed horizontally at 37°C for 1 h. The plate was washed with the washing solution three times and patted on absorbent papers to remove the remaining droplets as much as possible. 100 µL of TMB (Beijing Tiangen BioTech) was added to each well, and the plate was placed in the dark at room temperature for 45 min. 100 µL of 1 M H₂SO₄ terminating solution was added to each well to stop the substrate reaction, and the OD value at 450 nm was read with a microplate reader.

The results showed that B11 antibody can block the binding of hPD-L1 to hPD-1 and a dose-dependent manner was observed; as shown in Fig. 4.

### Example 7 The antibody blocks the interaction between CD80 and PD-L1

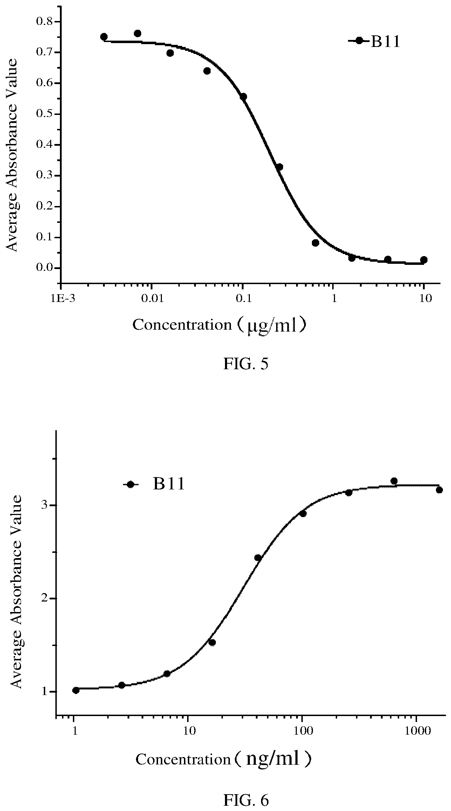

hPD-L1-Fc protein was diluted in coating buffer to a coating concentration of 1 µg/mL, added at 100 µl/well to the wells of the coating plate, and coating was performed overnight at 4°C. The plate was washed three times with washing solution (PBST), added with 2% bovine serum albumin in the corresponding well at 100 µL/well and blocked at 37°C for 2 h. B11 was 2.5-fold serial diluted to make a total of 10 concentrations with 10 µg/mL as the highest concentration. Biotin-labeled CD80 (CD80-Biotin) (R&D) was diluted with diluent (1% BSA) to 0.25 µg/mL, and the solution was mixed with the gradient-diluted B11 antibody at 1:1. The mixture was added to the blocked ELISA reaction wells, 180 µL/well, and triplicate wells were set for each sample concentration. The plate was covered with sealing membrane and placed horizontally at 37°C for 2 h. The plate was washed three times with the washing solution, added with HRP-labeled avidin (Shanghai Beyotime Biotech), and placed horizontally at 37°C for 1 h. The plate was washed with the washing solution three times and patted on absorbent papers to remove the remaining droplets as much as possible. 100 µL of TMB was added to each well, and the plate was placed in the dark at room temperature for 45 min. 100 µL of 1 M H₂SO₄ terminating solution was added to each well to stop the substrate reaction, and the OD value at 450 nm was read with a microplate reader. The activity of the antibody to block the interaction between CD80 and PD-L1 was analyzed. The results were shown in Fig. 5. B11 antibody can block the interaction between CD80 (CD80-Biotin) and PD-L1 and a dose-dependent manner was observed.

### Example 8 Cell biological activity of the antibody

CHO/PD-L1 cells (Promega) in logarithmic growth phase were taken and the cell density was adjusted to 5.0×10⁵ cells/mL with experimental medium (2% FBS). The cells were inoculated into a white opaque 96-well cell culture plate at 80 µL/well and incubated in CO₂ incubator for 16-18 h.

Experimental group: B11 was diluted to 6.4 µg/mL with RPMI1640 medium (containing 2% fetal bovine serum, all obtained from Gibco), diluted in 2.5-fold gradient to obtain a total of 9 concentration points, with triplicate wells for each sample concentration, and added at 40 µL/well to the culture plate inoculated with CHO/PD-L1 cells. Blank control group: 40 µL/well of experimental medium was added to the culture plate inoculated with CHO/PD-L1 cells. Jurkat/PD-1 (Promega) was adjusted to a cell density of 1.0×10⁶ - 1.25×10⁶ cells/mL and added at 40 µL/well to the experimental group and the blank control group. The culture plate was incubated in an incubator for 5.5-6.5 h. 80 µL of luciferase substrate solution (Promega) was added to each well. The reaction was performed in dark at room temperature for 5-30 min. The relative light units (RLU) were read through a microplate reader in chemiluminescence mode and the analysis software of the system was used for data analysis. The logarithm of the antibody concentration was the abscissa, the corresponding folds of signal induction (calculation formula: experimental well RLU/blank control RLU) was the ordinate, and the four-parameter equation curve was fitted.

The results showed that B11 antibody has cell biological activity and a dose dependent manner was observed. The results were shown in Fig. 6.

### Example 9 In vivo inhibitory activity of the antibody against colorectal cancer

In this example, the tumor-bearing model established by transplanting humanized B-hPD-1 mice subcutaneously with MC38-hPD-L1 colon cancer cells was used to evaluate the *in vivo* anti-tumor activity of the B11 antibody.

MC38-hPD-L1 colon cancer cells (CrownBio) were subcutaneously inoculated into the right anterior flank of female B-hPD-1 humanized mice (CrownBio) at 5×10⁵ cells/0.1 mL. When the tumors grew to about 160 mm³, they were randomly grouped into: solvent control, Tecentriq (10 mg/kg, Q2D×8), B11 (10 mg/kg, Q2D×8), B11 (3 mg/kg, Q2D×8), B11 (1 mg/kg, Q2D×8) according to tumor volume (10 in each group, 5 groups in total), respectively. The route of administration was intraperitoneal injection, and the experiment ended after the 22^{nd} day of group administration. The tumor volume and body weight were measured twice a week, and the mice body weight and tumor volume were recorded.

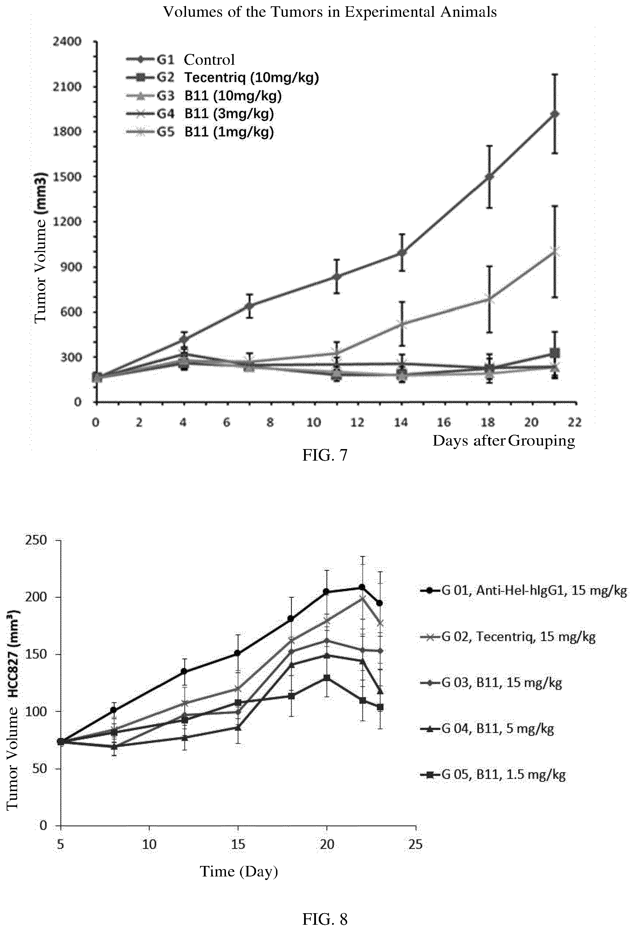

The results showed that during the experiment, the animals were kept in good health, and the mice were well tolerated to each therapeutic antibody. The results of tumor inhibition were shown in Fig. 7 and Table 2. At the end of the experiment (i.e. the 21^{st} day of group administration), the average tumor volume of the solvent control group was 1917±264 mm³. The average tumor volumes of the B11 group at the dose levels of 10 mg/kg, 3 mg/kg, 1 mg/kg were 232±76 mm³, 235±71 mm³ and 999±306 mm³, respectively; the tumor inhibition rates were 96.0%, 95.9% and 52.3%, respectively; and the tumor weight inhibition rates were 92.4%, 91.9% and 60.2% , respectively. The average tumor volume of the positive control drug Tecentriq (Roche) at a dose level of 10 mg/kg was 323±146 mm³, the tumor inhibition rate was 90.8%, and the tumor weight inhibition rate was 91.1%. Compared with the solvent control group, Tecentriq and B11 both showed significant difference in tumor volume (P<0.05), indicating that B11 has a significant tumor inhibitory effect.

At the dose of 3 mg/kg, the activity of anti-PD-Ll Antibody B11 was equivalent to the efficacy of the control drug Tecentriq of 10 mg/kg, indicating that anti-PD-Ll Antibody B11 has a better anti-tumor activity than Tecentriq.

**Table 2. Tumor inhibitory effect of test samples on tumor weight of MC38-hPD-L1 colon cancer transplanted B-hPD-1 humanized mice**

| **Group** | **Dosage (mg/kg)** | **Tumor weight (g)^{a}** | **Tumor weight inhibition rate IR_{TW}(%)** | ***P*^{b}** |
|---|---|---|---|---|
| 1.Sodium Chloride Injection | - | 1.957±0.391 | -- | -- |
| 2.Tecentriq | 10 | 0.174±0.101 | 91.1 | <0.001 |
| 3.B11 | 10 | 0.149±0.049 | 92.4 | <0.001 |
| 4.B11 | 3 | 0.158±0.060 | 91.9 | <0.001 |
| 5.B11 | 1 | 0.779±0.315 | 60.2 | 0.030 |

| | | | | |
|---|---|---|---|---|
| Note: a: mean ± standard error; b: Statistical comparison of tumor weights between the administration group and the solvent control group 22 days after group administration, t-test analysis. | | | | |

### Example 10 In vivo inhibitory activity of the antibody against non-small cell lung cancer

In this example, human HCC827 non-small cell lung cancer xenograft NCG mice (Sino Biological) were used to establish the tumor-bearing model to evaluate the anti-tumor activity of the B11 antibody *in vivo.*

HCC827 cells in logarithmic growth phase (Sino Biological) were harvested and NCG mice were inoculated subcutaneously on the right back on Day 0 at a dose of 5×10⁶/mice. 5 days after tumor cell inoculation (Day 5), mice with moderate tumor volume were selected and uniformly divided into 5 groups, each with 12 mice. Intraperitoneal transplantation of PBMC (1×10⁸/mL) was performed, 6 out of 12 mice in each group were inoculated with PBMC from donor #86, and the other 6 mice were inoculated with PBMC from donor #119. The cells were resuspended in PBS (0.1 ml inoculation volume). The experimental groups were B11 15 mg/kg, B11 5 mg/kg and B11 1.5 mg/kg groups, positive control Tecentriq 15 mg/kg group and negative control (Human IgG1) 15 mg/kg group. Intraperitoneal injection administration was performed 5, 8, 12, 15, 19 and 22 days after tumor cell inoculation, for a total of six administrations. The efficacy was evaluated according to the relative tumor inhibition rate (TGIRTV), and the safety was evaluated according to the change of animal weight and death.

The results showed that no drug treatment-related toxic reaction occurred in each treatment group within 23 days of tumor inoculation, and the tumor-bearing mice were well tolerated to the tested antibodies at the testing doses.

The anti-tumor results were shown in Fig. 8. The B11 (15 mg/kg) treatment group showed a significant tumor inhibitory effect 12 and 15 days after tumor cell inoculation, and the relative tumor inhibition rates TGIRTV (%) were 31% and 34%, respectively, showing statistically significant difference compared to the control group (p values were 0.018 and 0.016, respectively).

The B11 (5 mg/kg) treatment group showed a significant tumor inhibitory effect 12, 15, and 20 days after tumor cell inoculation in the PBMC-transplanted treatment group, and the relative tumor inhibition rates TGIRTV (%) were 44%, 43%, and 28%, respectively, showing statistically significant difference compared to the control group (p values were 0.001, 0.006 and 0.046, respectively).

The B11 (1.5 mg/kg) treatment group showed a significant tumor inhibitory effect 12, 15, 18, 20 and 22 days after tumor cell inoculation, and the relative tumor inhibition rates TGIRTV (%) were 35%, 30%, 39%, 38% and 49%, respectively, showing statistically significant difference compared to the control group (p values were 0.013, 0.031, 0.005, 0.003 and 0.002, respectively).

The positive drug Tecentriq (15 mg/kg) showed a small tumor inhibition effect 12 days after tumor cell inoculation, and the relative tumor inhibition rate TGIRTV (%) was 23%, which did not show statistically significant difference compared to the control group (p value 0.084).

The above results showed that Antibody B11 has excellent anti-tumor activity and the activity is superior over Tecentriq.

### Sequence Listing

**B10**

| SEQ ID NO | Name | Sequence |
|---|---|---|
| SEQ ID NO:1 | HCDR1 | DSWIH |
| SEQ ID NO:2 | HCDR2 | WISPFGGSTYYADSVKG |
| SEQ ID NO:3 | HCDR3 | RHWPGGFDY |
| SEQ ID NO:4 | VH | EVQLVESGGGLVQPGGSLRLSCAASGFTFSDSWIHW VRQAPGKGLEWVAWISPFGGSTYYADSVKGRFTISA DTSKNTAYLQMNSLRAEDTAVYYCARRHWPGGFDY WGQGTLVTVSS |
| SEQ ID NO:5 | LCDR1 | RASQDVSTAVA |
| SEQ ID NO:6 | LCDR2 | SASFLYS |
| SEQ ID NO:7 | LCDR3 | QQFIFHPAT |
| SEQ ID NO:8 | VL | DIQMTQSPSSLSASVGDRVTITCRASQDVSTAVAWYQ QKPGKAPKLLIYSASFLYSGVPSRFSGSGSGTDFTLTIS SLQPEDFATYYCQQFIFHPATFGQGTKVEIK |

**B11**

| | | |
|---|---|---|
| SEQ ID NO:9 | HCDR1 | ETWLH |
| SEQ ID NO:10 | HCDR2 | WVSPFGGSTYYADSVKG |
| SEQ ID NO:11 | HCDR3 | RHWPGGFDY |
| SEQ ID NO:12 | VH | |
| SEQ ID NO:13 | LCDR1 | RASQDVSTAVA |
| SEQ ID NO:14 | LCDR2 | SASFLYS |
| SEQ ID NO:15 | LCDR3 | QQFLYHPAT |
| SEQ ID NO:16 | VL | |

**H12**

| | | |
|---|---|---|
| SEQ ID NO:17 | HCDR1 | ESWLH |
| SEQ ID NO:18 | HCDR2 | WVTPYGGSTYYADSVKG |
| SEQ ID NO:19 | HCDR3 | RHWPGGFDY |
| SEQ ID NO:20 | VH | EVQLVESGGGLVQPGGSLRLSCAASGFTFSESWLH WVRQAPGKGLEWVAWVTPYGGSTYYADSVKGRFT ISADTSKNTAYLQMNSLRAEDTAVYYCARRHWPGG FDYWGQGTLVTVSS |
| SEQ ID NO:21 | LCDR1 | RASQDVSTAVA |
| SEQ ID NO:22 | LCDR2 | SASFLYS |
| SEQ ID NO:23 | LCDR3 | QQFLYHPAT |
| SEQ ID NO:24 | VL | |
| SEQ ID NO:25 | Light chain constant region | |
| SEQ ID NO:26 | Heavy chain constant region | ASTKGPSVFPLAPSSKSTSGGTAALGCLVKDYFPEPV TVSWNSGALTSGVHTFPAVLQSSGLYSLSSVVTVPS SSLGTQTYICNVNHKPSNTKVDKKVEPKSCDKTHTC PPCPAPELLGGPSVFLFPPKPKDTLMISRTPEVTCVV VDVSHEDPEVKFNWYVDGVEVHNAKTKPREEQYA STYRVVSVLTVLHQDWLNGKEYKCKVSNKALPAPI EKTISKAKGQPREPQVYTLPPSREEMTKNQVSLTCL VKGFYPSDIAVEWESNGQPENNYKTTPPVLDSDGSF FLYSKLTVDKSRWQQGNVFSCSVMHEALHNHYTQ KSLSLSPG |
| SEQ ID NO:27 | B11 Light chain full length | DIQMTQSPSSLSASVGDRVTITCRASQDVSTAVAWYQ QKPGKAPKLLIYSASFLYSGVPSRFSGSGSGTDFTLTIS SLQPEDFATYYCQQFLYHPATFGQGTKVEIKRTVAAP SVFIFPPSDEQLKSGTASVVCLLNNFYPREAKVQWKV DNALQSGNSQESVTEQDSKDSTYSLSSTLTLSKADYE KHKVYACEVTHQGLSSPVTKSFNRGEC |
| SEQ ID NO:28 | B11 Heavy chain full length | EVQLVESGGGLVQPGGSLRLSCAASGFTFSETWLHW VRQAPGKGLEWVAWVSPFGGSTYYADSVKGRFTISA DTSKNTAYLQMNSLRAEDTAVYYCARRHWPGGFDY WGQGTLVTVSSASTKGPSVFPLAPSSKSTSGGTAALG CLVKDYFPEPVTVSWNSGALTSGVHTFPAVLQSSGLY SLSSVVTVPSSSLGTQTYICNVNHKPSNTKVDKKVEP KSCDKTHTCPPCPAPELLGGPSVFLFPPKPKDTLMISR TPEVTCVVVDVSHEDPEVKFNWYVDGVEVHNAKTK PREEQYASTYRVVSVLTVLHQDWLNGKEYKCKVSN KALPAPIEKTISKAKGQPREPQVYTLPPSREEMTKNQ VSLTCLVKGFYPSDIAVEWESNGQPENNYKTTPPVLD SDGSFFLYSKLTVDKSRWQQGNVFSCSVMHEALHNH YTQKSLSLSPG |

A person skilled in the art will know that many modifications and changes of the invention can be made without departing from its spirit and scope. The specific embodiments described herein are only provided by way of examples and are not intended to be limited in any way. The actual scope and spirit are shown by the appended claims and the description and embodiments are only exemplary.

## Claims

1. An antibody against PD-L1 or an antigen-binding fragment thereof, the antibody or the antigen-binding fragment thereof specifically recognizing and binding to PD-L1, wherein the antibody is selected from the group consisting of:
Antibody B10,
wherein the Antibody B10 comprises a light chain variable region and a heavy chain variable region, wherein
the heavy chain variable region comprises:
H CDR1, comprising an amino acid sequence as shown in SEQ ID NO:1,
H CDR2, comprising an amino acid sequence as shown in SEQ ID NO:2, and
H CDR3, comprising an amino acid sequence as shown in SEQ ID NO:3;
the light chain variable region comprises:
L CDR1, comprising an amino acid sequence as shown in SEQ ID NO:5,
L CDR2, comprising an amino acid sequence as shown in SEQ ID NO:6, and
L CDR3, comprising an amino acid sequence as shown in SEQ ID NO:7;
Antibody B11,
wherein the Antibody B11 comprises a light chain variable region and a heavy chain variable region, wherein
the heavy chain variable region comprises:
H CDR1, comprising an amino acid sequence as shown in SEQ ID NO:9,
H CDR2, comprising an amino acid sequence as shown in SEQ ID NO:10, and
H CDR3, comprising an amino acid sequence as shown in SEQ ID NO:11;
the light chain variable region comprises:
L CDR1, comprising an amino acid sequence as shown in SEQ ID NO:13,
L CDR2, comprising an amino acid sequence as shown in SEQ ID NO:14, and
L CDR3, comprising an amino acid sequence as shown in SEQ ID NO:15;
Antibody H12,
wherein the Antibody H12 comprises a light chain variable region and a heavy chain variable region, wherein
the heavy chain variable region comprises:
H CDR1, comprising an amino acid sequence as shown in SEQ ID NO:17,
H CDR2, comprising an amino acid sequence as shown in SEQ ID NO:18, and
H CDR3, comprising an amino acid sequence as shown in SEQ ID NO:19;
the light chain variable region comprises:
L CDR1, comprising an amino acid sequence as shown in SEQ ID NO:21,
L CDR2, comprising an amino acid sequence as shown in SEQ ID NO:22, and
L CDR3, comprising an amino acid sequence as shown in SEQ ID NO:23.

2. The antibody or the antigen-binding fragment thereof according to claim 1, wherein the Antibody B10 comprises a heavy chain variable region (VH), wherein the heavy chain variable region comprises an amino acid sequence as shown in SEQ ID NO:4 or an amino acid sequence having at least 85%, at least 90%, at least 95% or more sequence identity to SEQ ID NO:4.

3. The antibody or the antigen-binding fragment thereof according to claim 1 or 2, wherein the Antibody B10 comprises a light chain variable region (VL), wherein the light chain variable region comprises an amino acid sequence as shown in SEQ ID NO:8 or an amino acid sequence having at least 85%, at least 90%, at least 95% or more sequence identity to SEQ ID NO:8.

4. The antibody or the antigen-binding fragment thereof according to claim 1, wherein the Antibody B11 comprises a heavy chain variable region (VH), wherein the heavy chain variable region comprises an amino acid sequence as shown in SEQ ID NO:12 or an amino acid sequence having at least 85%, at least 90%, at least 95% or more sequence identity to SEQ ID NO:12.

5. The antibody or the antigen-binding fragment thereof according to claim 1 or 4, wherein the Antibody B11 comprises a light chain variable region (VL), wherein the light chain variable region comprises an amino acid sequence as shown in SEQ ID NO:16 or an amino acid sequence having at least 85%, at least 90%, at least 95% or more sequence identity to SEQ ID NO:16.

6. The antibody or the antigen-binding fragment thereof according to any one of claims 1, 4 and 5, wherein the Antibody B11 comprises a heavy chain, wherein the heavy chain comprises an amino acid sequence as shown in SEQ ID NO:28 or an amino acid sequence having at least 85%, at least 90%, at least 95% or more sequence identity to SEQ ID NO:28.

7. The antibody or the antigen-binding fragment thereof according to any one of claims 1 and 4-6, wherein the Antibody B11 comprises a light chain, wherein the light chain comprises an amino acid sequence as shown in SEQ ID NO:27 or an amino acid sequence having at least 85%, at least 90%, at least 95% or more sequence identity to SEQ ID NO:27.

8. The antibody or the antigen-binding fragment thereof according to claim 1, wherein the Antibody H12 comprises a heavy chain variable region (VH), wherein the heavy chain variable region comprises an amino acid sequence as shown in SEQ ID NO:20 or an amino acid sequence having at least 85%, at least 90%, at least 95% or more sequence identity to SEQ ID NO:20.

9. The antibody or the antigen-binding fragment thereof according to claim 1 or 8, wherein the Antibody H12 comprises a light chain variable region (VL), wherein the light chain variable region comprises an amino acid sequence as shown in SEQ ID NO:24 or an amino acid sequence having at least 85%, at least 90%, at least 95% or more sequence identity to SEQ ID NO:24.

10. The antibody or the antigen-binding fragment thereof according to any one of claims 1-9, wherein, the Antibody B10, Antibody B11 or Antibody H12 comprises a heavy chain constant region (CH), wherein the heavy chain constant region comprises an amino acid sequence as shown in SEQ ID NO:26 or an amino acid sequence having at least 85%, at least 90%, at least 95% or more sequence identity to SEQ ID NO:26.

11. The antibody or the antigen-binding fragment thereof according to any one of claims 1-10, wherein, the Antibody B10, Antibody B11 or Antibody H12 comprises a light chain constant region (CL), wherein the light chain constant region comprises an amino acid sequence as shown in SEQ ID NO:25 or an amino acid sequence having at least 85%, at least 90%, at least 95% or more sequence identity to SEQ ID NO:25.

12. The antibody or the antigen-binding fragment thereof according to any one of claims 1-11, wherein the antibody or the antigen-binding fragment thereof is capable of specifically recognizing and binding to PD-L1, wherein the affinity (KD) is 1×10⁻⁹ M or less, preferably 5×10⁻¹⁰ M or less, more preferably 1×10⁻¹⁰ M or less.

13. The antibody or the antigen-binding fragment thereof according to any one of claims 1-12, wherein the antibody or the antigen-binding fragment thereof has at least one of the following:
1) blocking the interaction between PD-L1 and PD-1;
2) blocking the interaction between PD-L1 and CD80;
3) activating T cells;
4) inhibiting tumor growth.

14. The antibody or the antigen-binding fragment thereof according to any one of claims 1-13, wherein the antibody is human antibody.

15. A pharmaceutical composition, comprising the antibody or the antigen-binding fragment thereof according to any one of claims 1-14 or a combination thereof, and a pharmaceutically acceptable carrier.

16. Use of the antibody or the antigen-binding fragment thereof according to any one of claims 1-14 or the pharmaceutical composition according to claim 15 for the manufacture of a medicament for treating a neoplastic disease.

17. The use according to claim 16, wherein the neoplastic disease is a PD-L1-positive tumor.

18. The use according to claim 17, wherein the PD-L1-positive tumor is lung cancer, ovarian cancer, colorectal cancer, melanoma, renal cancer, bladder cancer, breast cancer, liver cancer, lymphoma, malignant hematological disease, head and neck cancer, glioma, stomach cancer, nasopharyngeal cancer, laryngeal cancer, cervical cancer, uterine corpus carcinoma or osteosarcoma.

19. An isolated nucleic acid molecule, encoding the antibody or the antigen-binding fragment thereof according to any one of claims 1-14.

20. An expression vector, comprising the nucleic acid molecule according to claim 19.

21. A host cell, which is transformed with the nucleic acid molecule according to claim 19 or the expression vector according to claim 20.

22. A process for preparing an antibody against PD-L1 or an antigen-binding fragment thereof, comprising,
(i) culturing the host cell according to claim 21 under a condition suitable for the expression of a nucleic acid molecule or an expression vector, and
(ii) isolating and purifying the antibody or the antigen-binding fragment thereof expressed by the nucleic acid molecule or the expression vector.


SEQUENCE LISTING

<110>  HISUN BIORAY PHARMACEUTICAL CO., LTD.

<120>  ANTIBODIES TO PROGRAMMED DEATH LIGAND (PD-L1) AND APPLICATION THEREOF

<130>  P2019TC745

<160>  28

<170>  PatentIn version 3.5

<210>  1
<211>  5
<212>  PRT
<213>  Artificial Sequence

<220>
<223>  HCDR1

<400>  1

Asp Ser Trp Ile His
1               5


<210>  2
<211>  17
<212>  PRT
<213>  Artificial Sequence

<220>
<223>  HCDR2

<400>  2

Trp Ile Ser Pro Phe Gly Gly Ser Thr Tyr Tyr Ala Asp Ser Val Lys
1               5                   10                  15


Gly



<210>  3
<211>  9
<212>  PRT
<213>  Artificial Sequence

<220>
<223>  HCDR3

<400>  3

Arg His Trp Pro Gly Gly Phe Asp Tyr
1               5


<210>  4
<211>  118
<212>  PRT
<213>  Artificial Sequence

<220>
<223>  VH

<400>  4

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Asp Ser
            20                  25                  30

Trp Ile His Trp Val Arg Gln Ala Pro Gly Lys Gly Leu Glu Trp Val
        35                  40                  45

Ala Trp Ile Ser Pro Phe Gly Gly Ser Thr Tyr Tyr Ala Asp Ser Val
    50                  55                  60

Lys Gly Arg Phe Thr Ile Ser Ala Asp Thr Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Ala Arg Arg His Trp Pro Gly Gly Phe Asp Tyr Trp Gly Gln Gly Thr
            100                 105                 110

Leu Val Thr Val Ser Ser
        115

<210>  5
<211>  11
<212>  PRT
<213>  Artificial Sequence

<220>
<223>  LCDR1

<400>  5

Arg Ala Ser Gln Asp Val Ser Thr Ala Val Ala
1               5                   10

<210>  6
<211>  7
<212>  PRT
<213>  Artificial Sequence

<220>
<223>  LCDR2

<400>  6

Ser Ala Ser Phe Leu Tyr Ser
1               5

<210>  7
<211>  9
<212>  PRT
<213>  Artificial Sequence

<220>
<223>  LCDR3

<400>  7

Gln Gln Phe Ile Phe His Pro Ala Thr
1               5


<210>  8
<211>  107
<212>  PRT
<213>  Artificial Sequence

<220>
<223>  VL

<400>  8

Asp Ile Gln Met Thr Gln Ser Pro Ser Ser Leu Ser Ala Ser Val Gly
1               5                   10                  15


Asp Arg Val Thr Ile Thr Cys Arg Ala Ser Gln Asp Val Ser Thr Ala
            20                  25                  30


Val Ala Trp Tyr Gln Gln Lys Pro Gly Lys Ala Pro Lys Leu Leu Ile
        35                  40                  45


Tyr Ser Ala Ser Phe Leu Tyr Ser Gly Val Pro Ser Arg Phe Ser Gly
    50                  55                  60


Ser Gly Ser Gly Thr Asp Phe Thr Leu Thr Ile Ser Ser Leu Gln Pro
65                  70                  75                  80


Glu Asp Phe Ala Thr Tyr Tyr Cys Gln Gln Phe Ile Phe His Pro Ala
                85                  90                  95


Thr Phe Gly Gln Gly Thr Lys Val Glu Ile Lys
            100                 105


<210>  9
<211>  5
<212>  PRT
<213>  Artificial Sequence

<220>
<223>  HCDR1

<400>  9

Glu Thr Trp Leu His
1               5

<210>  10
<211>  17
<212>  PRT
<213>  Artificial Sequence

<220>
<223>  HCDR2

<400>  10

Trp Val Ser Pro Phe Gly Gly Ser Thr Tyr Tyr Ala Asp Ser Val Lys
1               5                   10                  15

Gly

<210>  11
<211>  9
<212>  PRT
<213>  Artificial Sequence

<220>
<223>  HCDR3

<400>  11

Arg His Trp Pro Gly Gly Phe Asp Tyr
1               5

<210>  12
<211>  118
<212>  PRT
<213>  Artificial Sequence

<220>
<223>  VH

<400>  12

<210>  13
<211>  11
<212>  PRT
<213>  Artificial Sequence

<220>
<223>  LCDR1

<400>  13

Arg Ala Ser Gln Asp Val Ser Thr Ala Val Ala
1               5                   10

<210>  14
<211>  7
<212>  PRT
<213>  Artificial Sequence

<220>
<223>  LCDR2

<400>  14

Ser Ala Ser Phe Leu Tyr Ser
1               5

<210>  15
<211>  9
<212>  PRT
<213>  Artificial Sequence

<220>
<223>  LCDR3

<400>  15

Gln Gln Phe Leu Tyr His Pro Ala Thr
1               5

<210>  16
<211>  107
<212>  PRT
<213>  Artificial Sequence

<220>
<223>  VL

<400>  16


<210>  17
<211>  5
<212>  PRT
<213>  Artificial Sequence

<220>
<223>  HCDR1

<400>  17

Glu Ser Trp Leu His
1               5


<210>  18
<211>  17
<212>  PRT
<213>  Artificial Sequence

<220>
<223>  HCDR2

<400>  18

Trp Val Thr Pro Tyr Gly Gly Ser Thr Tyr Tyr Ala Asp Ser Val Lys
1               5                   10                  15

Gly

<210>  19
<211>  9
<212>  PRT
<213>  Artificial Sequence

<220>
<223>  HCDR3

<400>  19

Arg His Trp Pro Gly Gly Phe Asp Tyr
1               5


<210>  20
<211>  118
<212>  PRT
<213>  Artificial Sequence

<220>
<223>  VH

<400>  20

Glu Val Gln Leu Val Glu Ser Gly Gly Gly Leu Val Gln Pro Gly Gly
1               5                   10                  15


Ser Leu Arg Leu Ser Cys Ala Ala Ser Gly Phe Thr Phe Ser Glu Ser
            20                  25                  30


Trp Leu His Trp Val Arg Gln Ala Pro Gly Lys Gly Leu Glu Trp Val
        35                  40                  45


Ala Trp Val Thr Pro Tyr Gly Gly Ser Thr Tyr Tyr Ala Asp Ser Val
    50                  55                  60


Lys Gly Arg Phe Thr Ile Ser Ala Asp Thr Ser Lys Asn Thr Ala Tyr
65                  70                  75                  80


Leu Gln Met Asn Ser Leu Arg Ala Glu Asp Thr Ala Val Tyr Tyr Cys
                85                  90                  95


Ala Arg Arg His Trp Pro Gly Gly Phe Asp Tyr Trp Gly Gln Gly Thr
            100                 105                 110


Leu Val Thr Val Ser Ser
        115


<210>  21
<211>  11
<212>  PRT
<213>  Artificial Sequence

<220>
<223>  LCDR1

<400>  21

Arg Ala Ser Gln Asp Val Ser Thr Ala Val Ala
1               5                   10


<210>  22
<211>  7
<212>  PRT
<213>  Artificial Sequence

<220>
<223>  LCDR2

<400>  22

Ser Ala Ser Phe Leu Tyr Ser
1               5


<210>  23
<211>  9
<212>  PRT
<213>  Artificial Sequence

<220>
<223>  LCDR3

<400>  23

Gln Gln Phe Leu Tyr His Pro Ala Thr
1               5


<210>  24
<211>  107
<212>  PRT
<213>  Artificial Sequence

<220>
<223>  VL

<400>  24

<210>  25
<211>  107
<212>  PRT
<213>  Artificial Sequence

<220>
<223>  Light chain constant region

<400>  25

<210>  26
<211>  329
<212>  PRT
<213>  Artificial Sequence

<220>
<223>  Heavy chain constant region

<400>  26

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Val Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Ala Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Glu Glu
225                 230                 235                 240

Met Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly
                325

<210>  27
<211>  214
<212>  PRT
<213>  Artificial Sequence

<220>
<223>  B11 Light chain full length

<400>  27

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Val Ser Thr Ala
            20                  25                  30

Val Ala Trp Tyr Gln Gln Lys Pro Gly Lys Ala Pro Lys Leu Leu Ile
        35                  40                  45

Tyr Ser Ala Ser Phe Leu Tyr Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Phe Leu Tyr His Pro Ala
                85                  90                  95

Thr Phe Gly Gln Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  28
<211>  447
<212>  PRT
<213>  Artificial Sequence

<220>
<223>  B11 Heavy chain full length

<400>  28

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Glu Thr
            20                  25                  30

Trp Leu His Trp Val Arg Gln Ala Pro Gly Lys Gly Leu Glu Trp Val
        35                  40                  45

Ala Trp Val Ser Pro Phe Gly Gly Ser Thr Tyr Tyr Ala Asp Ser Val
    50                  55                  60

Lys Gly Arg Phe Thr Ile Ser Ala Asp Thr Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Ala Arg Arg His Trp Pro Gly Gly Phe Asp Tyr Trp Gly Gln Gly Thr
            100                 105                 110

Leu Val Thr Val Ser Ser Ala Ser Thr Lys Gly Pro Ser Val Phe Pro
        115                 120                 125

Leu Ala Pro Ser Ser Lys Ser Thr Ser Gly Gly Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Gln Thr Tyr Ile Cys Asn Val Asn His Lys Pro Ser
        195                 200                 205

Asn Thr Lys Val Asp Lys Lys Val Glu Pro Lys Ser Cys Asp Lys Thr
    210                 215                 220

His Thr Cys Pro Pro Cys Pro Ala Pro Glu Leu Leu Gly Gly Pro Ser
225                 230                 235                 240

Val Phe Leu Phe Pro Pro Lys Pro Lys Asp Thr Leu Met Ile Ser Arg
                245                 250                 255

Thr Pro Glu Val Thr Cys Val Val Val Asp Val Ser His Glu Asp Pro
            260                 265                 270

Glu Val Lys Phe Asn Trp Tyr Val Asp Gly Val Glu Val His Asn Ala
        275                 280                 285

Lys Thr Lys Pro Arg Glu Glu Gln Tyr Ala Ser Thr Tyr Arg Val Val
    290                 295                 300

Ser Val Leu Thr Val Leu His Gln Asp Trp Leu Asn Gly Lys Glu Tyr
305                 310                 315                 320

Lys Cys Lys Val Ser Asn Lys Ala Leu Pro Ala Pro Ile Glu Lys Thr
                325                 330                 335

Ile Ser Lys Ala Lys Gly Gln Pro Arg Glu Pro Gln Val Tyr Thr Leu
            340                 345                 350

Pro Pro Ser Arg Glu Glu Met Thr Lys Asn Gln Val Ser Leu Thr Cys
        355                 360                 365

Leu Val Lys Gly Phe Tyr Pro Ser Asp Ile Ala Val Glu Trp Glu Ser
    370                 375                 380

Asn Gly Gln Pro Glu Asn Asn Tyr Lys Thr Thr Pro Pro Val Leu Asp
385                 390                 395                 400

Ser Asp Gly Ser Phe Phe Leu Tyr Ser Lys Leu Thr Val Asp Lys Ser
                405                 410                 415

Arg Trp Gln Gln Gly Asn Val Phe Ser Cys Ser Val Met His Glu Ala
            420                 425                 430

Leu His Asn His Tyr Thr Gln Lys Ser Leu Ser Leu Ser Pro Gly
        435                 440                 445